# EUROPEAN PATENT APPLICATION

(11) **EP 4 238 992 A1**
(43) Date of publication of application: **06.09.2023**
(21) Application number: 22159491.4
(22) Date of filing: 01.03.2022
(51) Int. Cl.: C07K 16/28, C07K 16/46, A61P 35/00

(54) **TWO IN ONE - ANTIBODIES BINDING TO EGFR/PD-L1-DOUBLE POSITIVE CELLS**

(71) Applicant: Technische Universität Darmstadt, 64289 Darmstadt (DE)
(72) Inventor: KOLMAR, Harald, 64367 Mühltal (DE); HARWARDT, Julia, 64331 Weiterstadt (DE); BOGEN, Jan Patrick, 64295 Darmstadt (DE); CARRARA, Stefania, 64367 Mühltal (DE); ULITZKA, Michael, 64293 Darmstadt (DE)
(74) Representative: Fuchs Patentanwälte Partnerschaft mbB

(57) **Abstract**

The present invention is related to a bispecific monoclonal antibody or antigen-binding fragment, or a substantially similar variant thereof characterized in that at least one Fab-region binds specifically to epidermal growth factor receptor (EGFR) and programmed death-ligand 1 (PD-L1).

## Description

### Field of the Invention

Evolutionary pressure has selected antibodies as key immune molecules acting against foreign pathogens. The development of monoclonal antibody technology has allowed their widespread use in research, real-time diagnosis and treatment of multiple diseases, including cancer.

However, compared with hematologic malignancies, solid tumors have often proven to be relatively resistant to antibody-based therapies. In an attempt to improve the tumor-targeting efficacy of antibodies, new formats with modified, multivalent properties have been generated.

Initially, these formats imitated the structure of native IgG, creating mostly monospecific, bivalent antibodies. Recently, novel multivalent antibodies have been developed to maximize tumor targeting capabilities through enhanced biodistribution and functional affinity.

In an attempt to improve tumor penetration, recombinant antibodies with modified properties have been generated. Novel antibody formats, such as the single-chain Fv (scFv; 25-30 kDa), exhibit better pharmacokinetics than intact IgGs for tissue penetration, and enable binding specificity to be encoded by a single gene.

The covalent conjugation of small recombinant antibody molecules with poly(ethylene glycol) (PEG) - also known as 'PEGylation' - is a well-established procedure to improve serum half-life by increasing the molecular mass and the hydrodynamic radius. Another approach is the genetic fusion of antibody fragments to albumin or albumin-binding proteins. This strategy is based on the observation that albumin has a half-life similar to that of IgG and also utilizes FcRn-mediated recycling processes.

Alternatively, the use of bispecific antibodies to recruit serum IgG provides the Fc-associated effector functions and prolongs residence time in serum. Multimerization is a particularly appealing approach to optimize antibody size for the desired pharmacokinetics, and to maximize tumor uptake by enhancing the functional affinity (avidity) of antibody fragments.

One class of new antibodies are the so-called: "Two-in-One antibodies", which are antibodies in which each Fab arm can bind to two different antigens.

In this application we present a new method for generating avian-derived "Two-in-One antibodies" which possess a number of advantageous properties as well as a bispecific monoclonal antibody or antigen-binding fragment, or a substantially similar variant thereof characterized in that at least one Fab-region binds specifically to epidermal growth factor receptor (EGFR) and programmed death-ligand 1 (PD-L1).

### Background of the Invention

The IgG-like architecture of "Two-in-One antibodies" accounts for low immunogenicity and also circumvents laborious engineering and purification steps to facilitate correct chain pairing. In this application the identification of Two-in-One antibody by yeast surface display (YSD) screening of a chicken-derived immune library is described. The resulting antibody simultaneously targets the epidermal growth factor receptor (EGFR) and programmed death ligand 1 (PD-L1) at the same Fv fragment with two non-overlapping paratopes. The dual action Fabs are capable of inhibiting EGFR signalling by binding to dimerization domain II as well as blocking the PD-1/PD-L1 interaction. Furthermore, the Two-in-One antibody demonstrates specific cellular binding properties on EGFR/PD-L1 double positive tumor cells. The presented strategy obviates the need for any additional CDR engineering as described in the prior art and allows for obtaining bifunctional and bispecific antibodies upon animal immunization. Therefore, the disclosed method paves the way for further development of therapeutic antibodies derived from avian immunization with novel and tailor-made binding properties.

### Detailed Description

In a first embodiment, the present invention pertains to a bispecific monoclonal antibody or antigen-binding fragment, or a substantially similar variant thereof characterized in that at least one Fab-region binds specifically to epidermal growth factor receptor (EGFR) and programmed death-ligand 1 (PD-L1). In one embodiment the present invention pertains to the antibody "HCP-LCE" (heavy chain PD-L1 - light chain EGFR), however, also the "HCE-LCP" (heavy chain EGFR - light chain PD-L1)-variant is encompassed.

In one embodiment the bispecific monoclonal antibody or antigen-binding fragment, or a substantially similar variant thereof is preferred which binds specifically to at least one of the domains of epidermal growth factor receptor (EGFR) and to at least one of the domains of programmed death-ligand 1 (PD-L1).

The term "antibody", as used herein, is intended to refer any antigen binding molecule, in some embodiments to immunoglobulin molecules comprised of four polypeptide chains, two heavy (H) chains and two light (L) chains interconnected by disulfide bonds. Each heavy chain is comprised of a heavy chain variable region ("HCVR" or "VH") and a heavy chain constant region (comprised of domains CH1, CH2 and CH3). Each light chain is comprised of a light chain variable region ("LCVR or "VL") and a light chain constant region (CL). The VH and VL regions can be further subdivided into regions of hypervariability, termed "complementarity determining regions" (CDR), interspersed with regions that are more conserved, termed "framework regions" (FR). Each VH and VL is composed of three CDRs and four FRs, arranged from amino-terminus to carboxy-terminus in the following order: FR1, CDR1, FR2, CDR2, FR3, CDR3, FR4.

Substitution of up to one, up to two or up to three amino acid residues per CDR, of up to two, up to three or up to four amino acid residues within all three CDRs of one light or heavy chain or omission of one or more CDRs is also possible.

In one embodiment up to one, up to two or up to three amino acid residues are substituted in HCDR1.

In another embodiment up to one, up to two or up to three amino acid residues are substituted in H-CDR2. In yet another embodiment up to one, up to two or up to three amino acid residues are substituted in H-CDR3. In one embodiment up to one, up to two or up to three amino acid residues are substituted in L-CDR1. In another embodiment up to one, up to two or up to three amino acid residues are substituted in L-CDR2. In yet another embodiment up to one, up to two or up to three amino acid residues are substituted in L-CDR3.

Antibodies have been described in the scientific literature in which one or two CDRs can be dispensed with for binding.

If a CDR or amino acid residue(s) thereof is omitted, it is usually substituted with an amino acid occupying the corresponding position in another human antibody sequence or a consensus of such sequences.

In one embodiment up to one, up to two or up to three amino acid residues are omitted in HCDR1.

In another embodiment up to one, up to two or up to three amino acid residues are omitted in H-CDR2. In yet another embodiment up to one, up to two or up to three amino acid residues are omitted in H-CDR3. In one embodiment up to one, up to two or up to three amino acid residues are omitted in L-CDR1. In another embodiment up to one, up to two or up to three amino acid residues are omitted in L-CDR2. In yet another embodiment up to one, up to two or up to three amino acid residues are omitted in L-CDR3.

Positions for substitution within CDRs and amino acids to substitute can also be selected empirically.

Empirical substitutions can be conservative or non-conservative substitutions. In some embodiments also in other parts of the antibody or antigen-fragment thereof amino acids may be exchanged by conservative amino acid substitution. Such a sequence variant, which differs from another sequence only by conservative amino acid substitution(s), is called herein "substantially similar variant".

A "conservative amino acid substitution" is one in which an amino acid residue is substituted by another amino acid residue having a side chain (R group) with similar chemical properties (e.g., similar charge or hydrophobicity). In general, a conservative amino acid substitution will not substantially change the functional properties of a protein. In cases where two or more amino acid sequences differ from each other by conservative substitutions, the percent or degree of similarity may be adjusted upwards to correct for the conservative nature of the substitution. Means for making this adjustment are well known to those of skill in the art.

Examples of groups of amino acids that have side chains with similar chemical properties, and whose substitution for each other constitutes conservative substitutions, include 1) aliphatic side chains: glycine, alanine, valine, leucine and isoleucine; 2) aliphatic hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartate and glutamate, and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine.

Alternatively, a conservative replacement is any change having a positive value in the PAM250 log-likelihood matrix disclosed in Gonnet et al. (1992) Science 256: 1443 45. A "moderately conservative" replacement is any change having a nonnegative value in the PAM250 log-likelihood matrix.

Sequence similarity for polypeptides is typically measured using sequence analysis software. Protein analysis software matches similar sequences using measures of similarity assigned to various substitutions, deletions and other modifications, including conservative amino acid substitutions.

For instance, GCG software contains programs such as GAP and BESTFIT which can be used with default parameters to determine sequence homology or sequence identity between closely related polypeptides, such as homologous polypeptides from different species of organisms or between a wild type protein and a mutein thereof.

Polypeptide sequences also can be compared using FASTA with default or recommended parameters; a program in GCG Version 6.1. FASTA (e.g., FASTA2 and FASTA3) provides alignments and percent sequence identity of the regions of the best overlap between the query and search sequences (Pearson (2000) supra).

Another preferred algorithm when comparing a sequence of the invention to a database containing a large number of sequences from different organisms is the computer program BLAST, especially BLASTP or TBLASTN, using default parameters. See, e.g., Altschul et al. (1990) J. Mol. Biol. 215: 403 410 and (1997) Nucleic Acids Res. 25:3389 402.

The term "sequence identity" when referring to a nucleic acid or antigen-binding fragment thereof, indicates that, when optimally aligned with appropriate nucleotide insertions or deletions with another nucleic acid (or its complementary strand), there is nucleotide sequence identity in at least about 90%, and more preferably at least about 95%, at least about 96%, at least about 97%, at least about 98% or at least about 99% of the nucleotide bases, as measured by any well-known algorithm of sequence identity, such as FASTA, BLAST or GAP, as discussed.

As applied to polypeptides, the term "substantial similarity" or "substantially similar" means that two peptide sequences, when optimally aligned, such as by the programs GAP or BESTFIT using default gap weights, share at least 90% sequence identity, even more preferably at least 95%, at least 98% or at least 99% sequence identity. Preferably, residue positions which are not identical differ by conservative amino acid substitutions.

In some embodiments, the invention is a monoclonal antibody, a polyclonal antibody, a recombinant antibody, a human antibody, a humanized antibody, a chimeric antibody, a multispecific antibody, or an antigen-binding fragment thereof. In some embodiments, the isolated antigen binding protein is a Fab fragment, a Fab' fragment, a F(ab')2 fragment, a Fv fragment, a diabody, a nanobody or a single chain antibody molecule. In some embodiments, the isolated antigen binding protein is of the IgG1-, IgG2- IgG3- or IgG4-type. In some embodiments the antibody may be of IgA-, IgD-, IgE- or IgM-type.

In one embodiment the invention is a monoclonal antibody of IgG1-Type. In another embodiment the invention is a scFV-fragment.

The term "human antibody", as used herein, is intended to include antibodies having variable and constant regions derived from human germline immunoglobulin sequences. The human mAbs of the invention may include amino acid residues not encoded by human germline immunoglobulin sequences (e.g., mutations introduced by random or site-specific mutagenesis in vitro or by somatic mutation in vivo), for example in the CDRs.

The term "antibody fragment", as used herein, refers to one or more fragments of an antibody that retains the ability to specifically bind to S1-protein and/or RBD. An "antibody fragment" may include a Fab fragment, a F(ab')2 fragment, a Fv fragment (including scFv-fragments), a dAb fragment, a fragment containing a CDR, or an isolated CDR.

The term "specifically binds" or "binds specifically" or the like, means that an antibody or antigen-binding fragment thereof forms a complex with an antigen that is relatively stable under physiologic conditions. Specific binding can be characterized by an medium to high affinity equilibrium dissociation constant "K_{D}". The term "K_{D}", as used herein, is intended to refer to the equilibrium dissociation constant of a particular antibody-antigen interaction.

"High affinity" binding can be characterized by an equilibrium dissociation constant of 1×10⁻⁷ M or less, more preferably of 1×10⁻⁸ M or less, even more preferably of 1×10⁻⁹ M or less; most preferably of 1×10⁻¹⁰ M or less (e.g., a smaller KD denotes a tighter binding) as measured by surface plasmon resonance, e.g. BIACORE^{™}, biolayer interferometer (BLI), SPRi, solution-affinity ELISA. The term "medium affinity" can be characterized by an equilibrium dissociation constant of 1×10⁻³ M or less, preferably 1×10⁻⁴ M or less, more preferably of 1×10⁻⁵ M or less, more preferably of 1×10⁻⁶ M or less.

It is one finding of the present invention that antibodies with "medium affinity" for each target are preferred, since they reduce the chance of binding to cells which are not double-positive for both targets (EGFR and PD-L1), but only display one of the targets at the surface. As such side-effects of the inventive antibodies are reduced and the specificity for double-positive tumor cells is increased. Thus, in one embodiment it is preferred, that only by binding to both targets on the cell surface the affinity is high enough to elicit inhibition of EGFR and PD-L1 activity and/or to use the inventive antibodies for identification, prevention and/or treatment of a disease or condition in a patient.

Thus, in another aspect of the present invention the K_{D} for each single binding to each target (EGFR or PD-L1) is 2-fold, preferably 5-fold, preferably 10-fold, preferably 20-fold, preferably 50-fold, preferably 100-fold, preferably 200-fold, preferably 250-fold higher (i.e. shows less affinity) than the combined binding-K_{D} to both targets (EGFR and PD-L1) on one cell.

An isolated antibody or antigen-binding fragment thereof that specifically binds EGFR and/or PD-L1 may, however, still have cross-reactivity to other related antigens, such as the same molecules from other species. Methods for determining whether two molecules specifically bind are well known in the art and include, for example, equilibrium dialysis, surface plasmon resonance, and the like.

In some embodiments of the present invention, the antibodies or antigen-binding fragments of the invention bind preferably medium affinity to each single target (e.g. EGFR and PD-L1), resulting in a combined high affinity to cells displaying both targets.

The term "Bio-Layer Interferometry (BLI)", as used herein, refers a label-free technology for measuring biomolecular interactions. It is an optical analytical technique that analyzes the interference pattern of white light reflected from two surfaces: a layer of immobilized protein on the biosensor tip, and an internal reference layer. Any change in the number of molecules bound to the biosensor tip causes a shift in the interference pattern that can be measured in real-time.

The binding between a ligand immobilized on the biosensor tip surface and an analyte in solution produces an increase in optical thickness at the biosensor tip, which results in a wavelength shift, Δλ, which is a direct measure of the change in thickness of the biological layer. Interactions are measured in real time, providing the ability to monitor binding specificity, rates of association and dissociation, or concentration, with high precision and accuracy.

Only molecules binding specifically to or dissociating from the biosensor can shift the interference pattern and generate a response profile. Unbound molecules, changes in the refractive index of the surrounding medium, or changes in flow rate do not affect the interference pattern.

This is a unique characteristic of bio-layer interferometry and extends its capability to perform in crude samples used in applications for protein-protein interactions, quantitation, affinity, and kinetics.

BLI-systems are commercially available such as for example Octet^{®} QKe (Fortebio/Sartorius GmbH, Göttingen, Germany).

The term "surface plasmon resonance", as used herein, refers to an optical phenomenon that allows for the analysis of real-time biospecific interactions by detection of alterations in protein concentrations within a biosensor matrix, for example using the BIACORE^{™} system.

An "isolated antibody", as used herein, is intended to refer to an antibody or antigen-binding fragment thereof that is substantially free of other antibody or antigen-binding fragment thereof having different antigenic specificities (e.g., an isolated antibody that specifically binds EGFR and/or PD-L1 is substantially free of mAbs that specifically bind antigens other than EGFR and/or PD-L1).

The term "compete" when used in the context of the antibody or antigen-binding fragment thereof means competition between the reference antibody or antigen-binding fragment thereof in binding specifically to the target protein (also called "antigen", in the specific case of the invention this is EGFR and/or PD-L1) with other antigen binding molecule(s).

Such antigen-binding molecule may be selected from the list of: natural ligands to EGFR and/or PD-L1; other antibodies (e.g. cetuximab, panitumumab) or antigen-binding fragments thereof; diabodies, nanobodies, anticalins, scab-proteins, single-chain antibody variable regions, single-chain fragments variables (scFv), etc.

This competition may be determined by an assay in which the reference antibody or antigen binding fragment thereof (i.e. one of the antibodies mentioned hereinunder) prevents or inhibits (e.g., reduces) specific binding of EGFR and/or PD-L1 to another antigen-binding molecule, such as for example a ligand, a test-antibody or antigen-binding fragment thereof.

Numerous types of competitive binding assays can be used to determine if one antigen binding protein competes with another, for example:
- solid phase direct or indirect radioimmunoassay (RIA), solid phase direct or indirect enzyme immunoassay (EIA), sandwich;
- competition assay (see, e.g., Stahli et al., 1983, Methods in Enzymology 9:242-253);
- solid phase direct biotin-avidin EIA (see, e.g., Kirkland et al., 1986, J. Immunol. 137:3614-3619)
- solid phase direct labeled assay;
- solid phase direct labeled sandwich assay (see, e.g., Harlow and Lane, 1988, Antibodies, A Laboratory Manual, Cold Spring Harbor Press);
- solid phase direct label RIA using I-125 label (see, e.g., Morel et al., 1988, Molec. Immunol. 25:7-15);
- solid phase direct biotin-avidin EIA (see, e.g., Cheung, et al., 1990, Virology 176:546-552); and direct labeled RIA (Moldenhauer et al., 1990, Scand. J. Immunol. 32:77-82).

Competitive inhibition is measured by determining the amount of label bound to the solid surface or cells in the presence of the test antigen binding protein. Usually the antigen binding protein is present in excess.

Antigen binding proteins identified by competition assay (competing antigen-binding molecules) include antigen binding proteins binding specifically to the same epitope as the reference antigen binding proteins and antigen binding proteins binding specifically to an adjacent epitope sufficiently proximal to the epitope bound by the reference antigen binding protein for steric hindrance to occur.

Usually, when a competing antigen binding protein is present in excess, it will inhibit (e.g., reduce) specific binding of a reference antigen binding protein to a common antigen by at least 40%, at least 45%, at least 50%, at least 55%, at least 60%, at least 65%, at least 70% or at least 75% or more.

In some instances, binding is inhibited by at least 80%, at least 85%, at least 90%, at least 95%, or at least 97% or more.

In one embodiment binding is inhibited by at least 95%.

In a second embodiment, the present invention pertains to a bispecific monoclonal antibody or antigen-binding fragment or a substantially similar variant thereof, comprising at least one Fv-region binding specifically to epidermal growth factor receptor (EGFR) and programmed death-ligand 1 (PD-L1).

In further embodiments, the present invention pertains to a bispecific monoclonal antibody or antigen-binding fragment or a substantially similar variant thereof, wherein the paratopes, i.e. the CDR-regions binding to EGFR and PD-L1, on said antibody or antigen-binding fragment or a substantially similar variant thereof do not overlap.

The term "does not overlap" or "non-overlapping" refers generally to sequences of the protein which are at least not identical to each other, preferably separated regions of the protein. In case of non-overlapping paratopes it is meant that the sequences on the antibody responsible for binding to one target epitope are different from the sequences on the antibody responsible for binding to a second target epitope. Non-overlapping paratopes have the advantage that the binding of the antibody to one target epitope does not interfere significantly with the binding to a second target epitope. Thus, for example a one-armed antibody, FAB-fragment or Fv-fragment according to the present invention can still bind to at least two targets at the same time, preferably to PD-L1 and EGFR, allowing the combination with further binders to further targets as described hereinunder.

The position of CDRs within a VH or VL region are defined according to Kabat, et al., Sequences of Proteins of Immunological Interest, 5th Ed. Public Health Service, National Institutes of Health, Bethesda, MD (1991). In another embodiment, the method IMGT may be used to define the CDRs (Brochet, X. et al., Nucl. Acids Res. 36, W503-508 (2008). PMID: 18503082. Giudicelli, V., Brochet, X., Lefranc, M.-P., Cold Spring Harb Protoc. 2011 Jun 1; 2011 (6). pii: pdb.prot5633. doi: 10.1101/pdb.prot5633).

In further embodiments, the present invention pertains to a bispecific monoclonal antibody or antigen-binding fragment or a substantially similar variant thereof, capable of inhibiting EGFR-signalling and blocking the PD-1/PD-L1 interaction.

EGFR (ErbB-1/HER1) belongs to the receptor-family ErbB which comprises four receptors: EGFR (ErbB-1/HER1), ErbB-2 (Neu, HER2), ErbB-3 (HER3), and ErbB-4 (HER4). This receptor tyrosine kinase family (RTK) of proteins has an extracellular ligand-binding domain, hydrophobic transmembrane domain, and a cytoplasmic tyrosine kinase domain. ErbB receptors are activated by growth factors of EGF-family characterized by three disulphide-bonds that confer binding specificity. Additional structural motifs include immunoglobulin-like domains, heparin-binding sites and glycosylation sites. Typical ligands of EGFR are EGF, Transforming growth factor-α (TGF-α), Amphiregulin (AR), Betacellulin (BTC), Heparin-binding epidermal growth factor (HB-EGF) and Epiregulin (EPR).

Receptor activation is triggered by the cascade of events on cell membrane:
1. Ligand binding: Each ligand binds to the extracellular domain of cognate ErbB receptors.
2. Receptor dimerization: Ligand binding induces the formation of receptor homo or hetero dimers.
3. Activation of kinase domain: Autophosphorylation of key tyrosine residues within carboxy terminal tail activates the receptor and acts as docking sites for proteins with Src homology 2 (SH2) and phosphotyrosine binding domain (PTB) to trigger cellular signalling.

The term "specifically blocking and/or inhibiting EGFR and/or EGFR-signalling" as used herein, therefore refers to any inhibition of the above mentioned signalling cascade, preferably the inhibition of the dimerization of EGFR (step 2).

Programmed cell death protein 1 (PD-1) plays a vital role in inhibiting immune responses and promoting self-tolerance through modulating the activity of T-cells, activating apoptosis of antigen-specific T-cells and inhibiting apoptosis of regulatory T-cells. Programmed cell death-ligand 1 (PD-L1) is a trans-membrane protein that is considered to be a co-inhibitory factor of the immune response, it can combine with PD-1 to reduce the proliferation of PD-1 positive cells, inhibit their cytokine secretion and induce apoptosis. PD-L1 also plays an important role in various malignancies where it can attenuate the host immune response to tumor cells. Based on these perspectives, PD-1/PD-L1 axis is responsible for cancer immune escape and makes a huge effect on cancer therapy.

The term "specifically blocking and/or inhibiting PD-L1 and/or PD-L1-signalling" as used herein, therefore refers to any inhibition of the above mentioned PD-1/PD-L1 signalling cascade, preferably the inhibition by binding to an at least partially overlapping epitope with durvalumab (MEDI4736).

In further embodiments, the present invention pertains to a bispecific monoclonal antibody or antigen-binding fragment or a substantially similar variant thereof, wherein the EGFR binding is at the dimerization domain II of EGFR and wherein the antibody prevents PD-1 binding and/or competes for binding to PD-L1 with durvalumab.

The term "epitope" is a region of an antigen that is bound by an antibody. Epitopes may be defined as structural or functional. Functional epitopes are generally a subset of the structural epitopes and have those residues that directly contribute to the affinity of the interaction.

Epitopes may also be conformational, that is, composed of non-linear amino acids. In certain embodiments, epitopes may include determinants that are chemically active surface groupings of molecules such as amino acids, sugar side chains, phosphoryl groups, or sulfonyl groups, and, in certain embodiments, may have specific three-dimensional structural characteristics, and/or specific charge characteristics.

In further embodiments, the present invention pertains to a bispecific monoclonal antibody or antigen-binding fragment or a substantially similar variant thereof, in one embodiment at least 90%, preferably at least 95%, most preferably at least 99% sequence identiy, comprising
a heavy chain CDR1 (H-CDR1) domain with an amino acid sequence selected from the group consisting of SEQ ID No.: 03; and/or
a heavy chain CDR2 (H-CDR2) domain with an amino acid sequence selected from the group consisting of SEQ ID No.: 04; and/or
a heavy chain CDR3 (H-CDR3) domain with an amino acid sequence selected from the group consisting of SEQ ID No.: 05; and/or
   and/or
a light chain CDR1 (L-CDR1) domain with an amino acid sequence selected from the group consisting of SEQ ID No.: 06; and/or
a light chain CDR2 (L-CDR2) domain with an amino acid sequence selected from the group consisting of SEQ ID No.: 07; and/or
a light chain CDR3 (L-CDR3) domain with an amino acid sequence selected from the group consisting of SEQ ID No.: 08.

In yet a further embodiment, the present invention pertains to a bispecific monoclonal antibody or antigen-binding fragment or a substantially similar variant thereof, in one embodiment at least 90%, preferably at least 95%, most preferably at least 99% sequence identity, comprising
a heavy chain CDR1 (H-CDR1) domain with an amino acid sequence selected from the group consisting of SEQ ID No.: 03; and
a heavy chain CDR2 (H-CDR2) domain with an amino acid sequence selected from the group consisting of SEQ ID No.: 04; and
a heavy chain CDR3 (H-CDR3) domain with an amino acid sequence selected from the group consisting of SEQ ID No.: 05; and
   and/or
a light chain CDR1 (L-CDR1) domain with an amino acid sequence selected from the group consisting of SEQ ID No.: 06; and
a light chain CDR2 (L-CDR2) domain with an amino acid sequence selected from the group consisting of SEQ ID No.: 07; and
a light chain CDR3 (L-CDR3) domain with an amino acid sequence selected from the group consisting of SEQ ID No.: 08.

In yet a further embodiment, the present invention pertains to a bispecific monoclonal antibody or antigen-binding fragment or a substantially similar variant thereof, in one embodiment at least 90%, preferably at least 95%, most preferably at least 99% sequence identity, comprising
a heavy chain CDR1 (H-CDR1) domain with an amino acid sequence selected from the group consisting of SEQ ID No.: 03; and
a heavy chain CDR2 (H-CDR2) domain with an amino acid sequence selected from the group consisting of SEQ ID No.: 04; and
a heavy chain CDR3 (H-CDR3) domain with an amino acid sequence selected from the group consisting of SEQ ID No.: 05; and
   and
a light chain CDR1 (L-CDR1) domain with an amino acid sequence selected from the group consisting of SEQ ID No.: 06; and
a light chain CDR2 (L-CDR2) domain with an amino acid sequence selected from the group consisting of SEQ ID No.: 07; and
a light chain CDR3 (L-CDR3) domain with an amino acid sequence selected from the group consisting of SEQ ID No.: 08.

In further embodiments, the present invention pertains to a bispecific monoclonal antibody or antigen-binding fragment or a substantially similar variant thereof, in one embodiment having at least 90%, preferably at least 95%, most preferably at least 99% sequence identity, comprising
a variable heavy chain VH-region with an amino acid sequence selected from the group consisting of SEQ ID No.: 01;
   and/or
a variable light chain VL-region domain with an amino acid sequence selected from the group consisting of SEQ ID No.: 02.

In further embodiments, the present invention pertains to a bispecific monoclonal antibody or antigen-binding fragment or a substantially similar variant thereof, in one embodiment having at least 90%, preferably at least 95%, most preferably at least 99% sequence identity, comprising a variable heavy chain region of the amino acid sequence in SEQ ID No.: 01 and a variable light chain region of the amino acid sequence in SEQ ID No.: 02.

In further embodiments, the present invention pertains to a bispecific monoclonal antibody or antigen-binding fragment or a substantially similar variant thereof, comprising an amino acid sequences at least 95% identical to the amino acid sequences of SEQ ID No.: 01 and/or of SEQ ID No.: 02.

In further embodiments, the present invention pertains to a bispecific monoclonal antibody or antigen-binding fragment or a substantially similar variant thereof, characterized by one or more of the following:
- human or humanized, preferably grafted into a human IgG1-scaffold;
- also capable of binding to at least a third target protein selected from the group comprising Fcγ-receptor IIIa /IIIb (CD16a/b), cluster of differentiation 155 (CD155), also known as the poliovirus receptor, MHC class I chain-related proteins A and B (MicA/MicB), cluster of differentiation 159 (CD159), a NKG2 receptor for natural killer cells (NK cells) (there are 7 NKG2 types: A, B, C, D, E, F and H; NKG2D is an activating receptor on the NK cell surface, NKG2A dimerizes with CD94 to make an inhibitory receptor), cluster of differentiation 276 (CD276) or B7 homolog 3 (B7-H3), hepatitis A virus cellular receptor 2 (HAVCR2), also known as T-cell immunoglobulin and mucin-domain containing-3 (TIM-3), T-cell immuno-receptor with Ig and ITIM domains (TIGIT), B-cell maturation antigen (BCMA or BCM), also known as tumor necrosis factor receptor superfamily member 17 (TNFRSF17), interleukin-3 receptor (CD123), natural cytotoxicity triggering receptor 3 (NKp30) or (CD337), natural cytotoxicity triggering receptor 3 (NKp44) or (CD336), natural cytotoxicity triggering receptor 3 (NKp46) or (CD335), cluster of differentiation 27 (CD27), cluster of differentiation 96 (CD96) or Tactile (T-cell activation, increased late expression), NKRP1A, cluster of differentiation 355 (CD355) or Cytotoxic and regulatory T-cell molecule (CRTAM), and lymphocyte function-associated antigen 1 (LFA-1), and/or any combination thereof;
- capable of binding specifically to PD-L1-protein with "medium affinity" i.e. an affinity (KD) of 500 nM or less, preferably of 350 nM or less, more preferably of 250 nM or less, even more preferably of 100 nM or less; in some embodiments between 0.1 and 500 nM, preferably between 1 and 450 nM, preferably between 5 and 250 nM, more preferably between 10 and 150 nM, even more preferably between 50 and 130 nM; in some embodiments about 78.3 nM, in some embodiments about 79.5 nM, in some embodiments about 83 nM, in some embodiments about 97.6 nM, in some embodiments about 104 nM, in some embodiments about 115 nM, in some embodiments about 125 nM, in some embodiments about 127 nM.

- capable of binding specifically to EGFR-protein with "medium affinity" i.e. an affinity (KD) of 1000 nM or less, more preferably 800 nM or less; more preferably 700 nM or less; in some embodiments between 50 and 1000 nM, preferably between 100 and 800 nM, more preferably between 150 and 500 nM, even more preferably between 200 and 350 nM; in some embodiments about 236 nM, in some embodiments about 294 nM, in some embodiments about 460 nM, in some embodiments about 570 nM, in some embodiments about 603 nM, in some embodiments about 763 nM, in some embodiments about 982 nM;
- capable to specifically bind to a tumour cell, wherein the tumour cell is characterized by being double-positive for PD-L1 and EGFR;
- inhibits the dimerization of EGFR, but not the binding of EGF to EGFR;
- inhibits EGFR-pathway signalling at least 2-fold or more, preferably between 3-fold and 10-fold, preferably between 3-fold and 8-fold, preferably between 3-fold and 5-fold; in some embodiments the IC₅₀ for inhibiting the EGFR-pathway is between 1 and 10 nm, preferably about 6.7 nM.
- inhibits the binding of PD-L1 to PD-1 with an EC₅₀ of between 1 and 1000 nM, preferably of between 5 and 500 nM, preferably of between 50 and 250 nM, preferably of about 214 nM;
- binds to double-positive cells (such as A431-cells) with EGFR and PD-L1 with "high affinity", i.e. an "effective concentration of binding" an EC₅₀ of between 0.1 and 50 nM, of between 0.5 and 20 nM, preferably between 1 and 10 nM, preferably between 1.25 and 5 nM, preferably of about 1.37 nM;
- binds to EGFR-positive cells with an EC₅₀ of between 5 and 50 nM, of between 10 and 20 nM, preferably of about 17.83 nM.
- being further modified to an antibody-drug conjugate (ADC) by being conjugated to a therapeutic agent, such as drug, a tumour-growth inhibitor, an apoptotic signal molecule, a toxin or the like;
- being further modified to extend its half-life by albumin-fusion, polyaminoacid fusion, Fc-fusion, or a polymer-conjugation with polyethylene glycol, sialic acid, starch and/or hyaluronic acid.

The half maximal inhibitory concentration (IC₅₀) is a measure of the potency of a substance in inhibiting a specific biological or biochemical function. IC₅₀ is a quantitative measure that indicates how much of a particular inhibitory substance (e.g. the inventive antibody) is needed to inhibit, in vitro, a given biological process or biological component by 50%. The biological component could be an enzyme, cell, cell receptor or microorganism, in the particular case of this invention it is EGFR and PD-L1-activity. IC₅₀ values are typically expressed as molar concentration.

IC₅₀ is commonly used as a measure of antagonist drug potency in pharmacological research. IC₅₀ is comparable to other measures of potency, such as EC₅₀ for excitatory drugs. EC₅₀ represents the dose or plasma concentration required for obtaining 50% of a maximum effect in vivo.

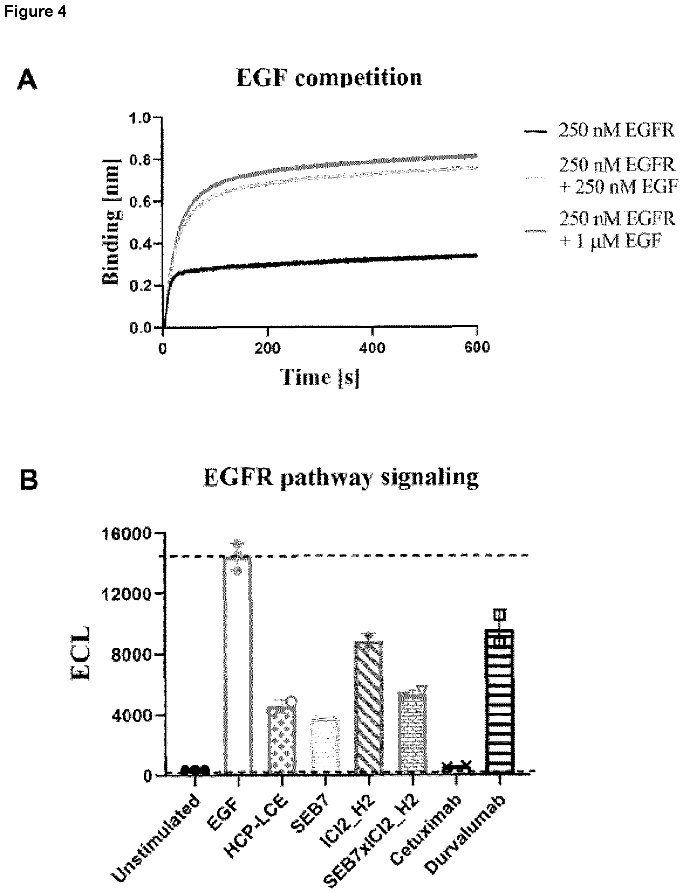

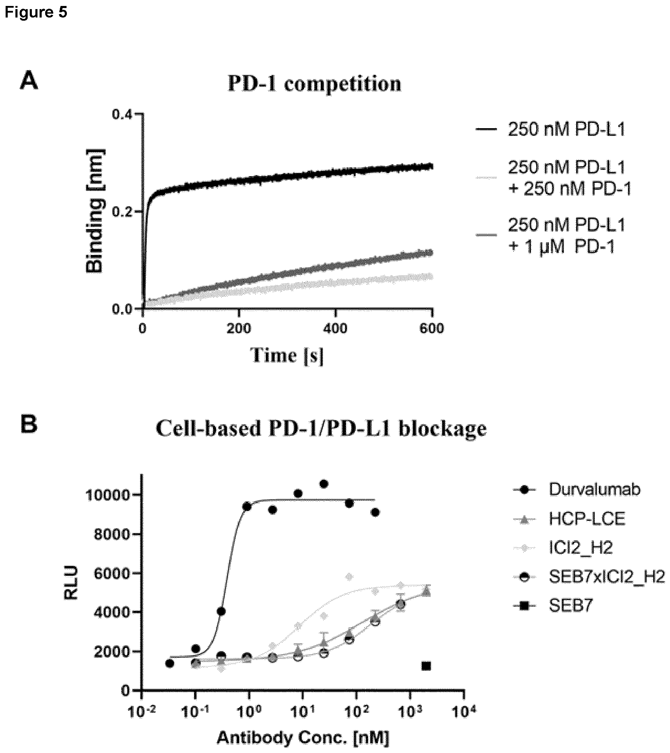

IC₅₀ can be determined with functional assays or with competition binding assays. In general, the Two-in-One antibody is loaded onto BLI biosensors and association is measured against the antigen, pre-incubated with its ligand or receptor. In the case that the antibody can still bind to the antigen despite binding of the natural ligand/receptor, it does not inhibit the interaction of the antigen with its ligand/receptor. If no binding is observed, the antibody binds an epitope that overlaps with the ligand/receptor (see also example section and figures 4A and 5A).

In another aspect of the present invention variants based on the inventive Two-in-One antibody HCP-LCE were produced. First of all a one-armed (oa) variant of the inventive antibody was produced to show its binding abilities and usefulness for further applications, such as scFv or Fab-fragments.

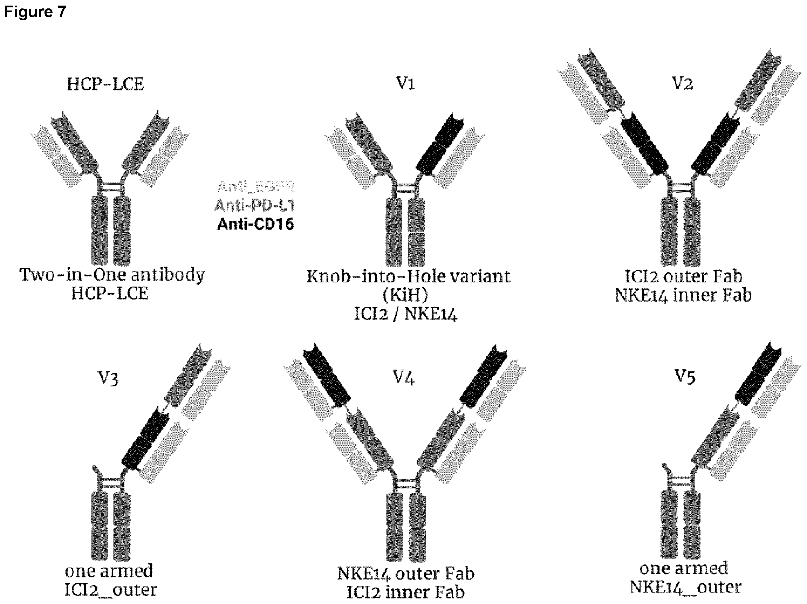

Further, as also depicted in Figure 7 and further described in the examples, five different trispecific variants were designed (cf. Figure 7, "V1" - "V5"):
1. A "Knob-into-Hole" (KiH) variant (cf. Figure 7, "V1") consisting of HCP-LCE on one side and the heavy chain of the CD16 binder (NKE14), paired with the HCP-LCE light chain.
2. Two variants with two Fabs (see KiH variant) each side in both possible orientations (cf. Figure 7, "V2" and "V4"):
   - ICI2_outer and NKE14_inner
   - NKE14_outer and ICI2_inner
3. both variants were also designed and produced as one-armed (oa) variants (cf. Figure 7, "V3" and "V5").

Thus, the present invention also pertains to trivalent and/or multivalent variants, which comprise besides binding-sites to EGFR and PD-L1 at least one further binding-site, able to bind for example to CD16.

Alternative and/or further targets may be selected from the group consisting of cluster of differentiation 155 (CD155), also known as the poliovirus receptor, MHC class I chain-related proteins A and B (MicA/MicB), cluster of differentiation 159 (CD159), a NKG2 receptor for natural killer cells (NK cells) (there are 7 NKG2 types: A, B, C, D, E, F and H; NKG2D is an activating receptor on the NK cell surface, NKG2A dimerizes with CD94 to make an inhibitory receptor), cluster of differentiation 276 (CD276) or B7 homolog 3 (B7-H3), hepatitis A virus cellular receptor 2 (HAVCR2), also known as T-cell immunoglobulin and mucin-domain containing-3 (TIM-3), T-cell immuno-receptor with Ig and ITIM domains (TIGIT), B-cell maturation antigen (BCMA or BCM), also known as tumor necrosis factor receptor superfamily member 17 (TNFRSF17), interleukin-3 receptor (CD123), natural cytotoxicity triggering receptor 3 (NKp30) or (CD337), natural cytotoxicity triggering receptor 3 (NKp44) or (CD336), natural cytotoxicity triggering receptor 3 (NKp46) or (CD335), cluster of differentiation 27 (CD27), cluster of differentiation 96 (CD96) or Tactile (T-cell activation, increased late expression), NKRP1A, cluster of differentiation 355 (CD355) or Cytotoxic and regulatory T-cell molecule (CRTAM), and lymphocyte function-associated antigen 1 (LFA-1), and/or any combination thereof.

The binding kinetics as measured by BLI for the third or further target site should be in the range of between 5 nM and 1000 nM, preferably between 50 nM and 800 nM, more preferably between 100 nM and 750 nM, even more preferably between 200 nM and 500 nM, even more preferably between 250 nM and 390 nM, in some embodiments between 270 - 280 nM or about 277 nM, between 295 - 310 nM or about 302 nM, between 335 - 352 nM or about 342 nM, between 375 - 392 nM or about 382 nM.

In further embodiments, the present invention pertains to an isolated nucleic acid molecule encoding the bispecific monoclonal antibody or antigen-binding fragment or a substantially similar variant thereof.

In further embodiments, the present invention pertains to an expression vector comprising the before mentioned nucleic acid molecule.

In yet another aspect the invention pertains to a method of producing the antibody according to the invention, preferably a HCP-LCE- or HCE-LCP-antibody, or antigen-binding fragment or a substantially similar variant thereof comprising the steps of
i) Selecting a first and a second human target protein, preferably represented on the surface of tumour cells, preferably EGFR and PD-L1;
ii) Creating a first yeast surface display (YSD) library, comprising the following steps:
   a. Immunizing an animal of avian species, preferably a chicken, with the first target protein;
   b. Isolating variable heavy chain (VH) genes from cDNA derived from said immunized animal of avian species;
   c. Inserting the VH genes into a vector designed for expression, secretion, and display of proteins on the extracellular surface of *Saccharomyces cerevisiae* cells , preferably pYD1-derived, and transform yeast cells with said vector; in one embodiment said vector further comprises a constant heavy chain (CH1), glutamine synthetase (GS) and/or Aga2p (from *Saccharomyces cerevisiae*).
   d. Inserting genes for a common variable light chain (VL) (SEQ ID No.: 16) into an expression vector designed for expression, secretion, and display of proteins on the extracellular surface of *Saccharomyces cerevisiae* cells, preferably pYD1-derived and transform yeast cells with said vector; in one embodiment said vector further comprises a constant light chain (CL1);
   e. Combining the two yeast cells by yeast cell mating, resulting in diploid yeast cells displaying VH domains from the PD-L1-immunized animal of avian species, paired with the common variable light chain (VL) (SEQ ID No.: 16);
   f. Screening by fluorescence activated cell sorting (FACS) for the binding to the first target protein and isolate yeast cells displaying antibodies which specifically bind to the first target protein;
iii) Creating a second yeast surface display (YSD) library, comprising the following steps:
   a. Immunizing an animal of avian species, preferably a chicken, with the second target protein, preferably EGFR if the first antigen was PD-L1; or PD-L1 if the first antigen was EGFR;
   b. Isolating variable light chain (VL) genes from cDNA derived from said immunized animal of avian species;
   c. Inserting the common variable heavy chain (VH) genes (SEQ ID No.: 15) as described in the previous screen into an vector designed for expression, secretion, and display of proteins on the extracellular surface of *Saccharomyces cerevisiae* cells, preferably pYD1-derived, and transform yeast cells with said vector;
iv) Combining the yeast cells from the first YSD-library carrying heavy chains (VH) specifically binding to the first target protein with the second YSD-library carrying the light chains (VL) from the animal of avian species immunized with the second target protein, by yeast mating
v) Screening the resulting yeast cells by fluorescence activated cell sorting (FACS) for binding to both target proteins and/or inhibition of each of the two target proteins;
vi) Identifying the yeast cells comprising antibodies comprising heavy chains specifically binding to the first target protein and comprising light chains specifically binding to the second target protein;
vii)lsolating the bispecific, tetravalent monoclonal antibody or antigen-binding fragment binding to both target proteins (in one embodiment humanized and/or human), preferably EGFR and PD-L1.

Since each arm of the inventive antibodies, as produced by the before mentioned method, binds to both targets with at least medium affinity, it may be combined with another "arm" binding to at least one, at least two or more further targets. This is also exemplified in the example section, where the HCP-LCE-antibody according to the invention was combined with a CD16-binding entity (NKE14). Thus, this invention also encompasses methods to produce antibodies binding to one, two or more targets besides binding to EGFR and PD-L1.

The described method relies on screening of combinational immune-libraries and obviates the need for any additional CDR engineering as described in the prior art. Thus, this invention paves the way for further development of therapeutic antibodies derived from avian immunization with novel and tailor-made binding properties.

Thus, one advantage of the inventive method is that it allows for the isolation of chicken-derived Two-in-One antibodies without CDR engineering by combining the heavy chain of an anti-PD-L1 common light chain antibody with an anti-EGFR immune light chain library and YSD screening. The resulting antibody HCP-LCE simultaneously targets EGFR and PD-L1 at the same Fv fragment while exhibiting favorable biophysical properties and aggregation behaviour. The Two-in-One antibody is able to inhibit EGFR signaling by binding to dimerization domain II and to block the PD-1/PD-L1 interaction. Furthermore, HCP-LCE demonstrated specific cellular binding properties on EGFR/PD-L1 double-positive tumor cells. The inventive antibodies are the first Two-in-One antibodies without CDR engineering that simultaneously target two antigens with the same Fab fragment.

The invention further provides therapeutic compositions comprising the antibodies or antigen-binding fragments thereof of the present invention. The administration of therapeutic compositions in accordance with the invention will be administered with suitable carriers, excipients, and other agents that are incorporated into formulations to provide improved transfer, delivery, tolerance, and the like.

A multitude of appropriate formulations can be found in the formulary known to all pharmaceutical chemists: Remington's Pharmaceutical Sciences, Mack Publishing Company, Easton, PA.

These formulations include, for example, aqueous solutions, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax.

The dose may vary depending upon the age and the size of a subject to be administered, severity of the target disease, individual condition of the subject (for example in case of a kidney-disease the dosage may be reduced to between 25%-75% as compared to the dosage for healthy patients of the same gender and weight), route of administration, and the like.

Depending on the severity of the condition, the frequency and the duration of the treatment can be adjusted.

Various delivery systems are known and can be used to administer the pharmaceutical composition of the invention, e.g., encapsulation in liposomes, microparticles, microcapsules, recombinant cells capable of expressing the mutant viruses, receptor mediated endocytosis.

Methods of introduction include, but are not limited to, intradermal, intramuscular, intraperitoneal, intravenous, subcutaneous, intranasal, epidural, inhalative and oral routes; for example as full-length, fragment, FcRn, EPO-Fc, RNA- or DNA-precursor, or the like.

The composition may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

The pharmaceutical composition can be also delivered in a vesicle, in particular a liposome. In certain embodiments, the pharmaceutical composition can be delivered in a controlled release system, such as a pre-filled syringe, a dermal patch, infusion (for example in case of Bi-specific T-cell engager- (BiTE)-antibodies), pen- or auto-injector-device (single-dose, multi-dose, disposable, re-usable), syrette, large-volume pump-device, and the like.

In one embodiment a pre-filled syringe, or auto-injector, or pump device is preferred.

In another embodiment, polymeric materials can be used. In yet another embodiment, a controlled release system can be placed in proximity of the composition's target, thus requiring only a fraction of the systemic dose.

The injectable preparations may include dosage forms for intravenous, subcutaneous, intracutaneous and intramuscular injections, drip infusions, etc.

These injectable preparations may be prepared by methods publicly known.

For example, the injectable preparations may be prepared, e.g., by dissolving, suspending or emulsifying the antibody or its salt described above in a sterile aqueous medium or an oily medium conventionally used for injections.

As the aqueous solution for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc.

As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

A pharmaceutical composition of the present invention can be delivered subcutaneously or intravenously with a standard needle and syringe.

In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused.

In a disposable pen delivery device, there is no replaceable cartridge. Rather, the disposable pen delivery device comes prefilled with the pharmaceutical composition held in a reservoir within the device.

Once the reservoir is emptied of the composition, the entire device is discarded.

Numerous reusable pen and auto-injection delivery devices have applications in the subcutaneous delivery of a pharmaceutical composition of the present invention. Examples include, but certainly are not limited to AUTOPEN^{™} (Owen Mumford, Inc., Woodstock, UK), DISETRONIC^{™} pen (Disetronic Medical Systems, Burghdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™}I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™} OPTIPEN STARLET^{™}, and OPTICLIK^{™} (sanofi-aventis, Frankfurt, Germany), to name only a few.

Examples of disposable pen delivery devices having applications in subcutaneous delivery of a pharmaceutical composition of the present invention include, but certainly are not limited to the SOLOSTAR^{™} pen (sanofi-aventis), the FLEXPEN^{™} (Novo Nordisk), and the KWIKPEN^{™} (Eli Lilly).

Advantageously, the pharmaceutical compositions for oral or parenteral use described above are prepared into dosage forms in a unit dose suited to fit a dose of the active ingredients.

Such dosage forms in a unit dose include, for example, tablets, pills, capsules, injections (ampoules), suppositories, etc.

The amount of the aforesaid antibody contained is generally about 5 to about 500 mg per dosage form in a unit dose; especially in the form of injection, it is preferred that the aforesaid antibody is contained in about 5 to about 100 mg and in about 10 to about 250 mg for the other dosage forms.

In further embodiments, the present invention pertains to a bispecific monoclonal antibody or antigen-binding fragment, or a substantially similar variant thereof for the use in the identification, prevention and/or treatment of a disease or condition related to cancer in a patient.

In particular the inventive antibodies can be used for identification, prevention and/or treatment of PD-L1-positive tumors, such as non-small cell lung carcinomas, malignant melanomas, bladder carcinomas, Hodgkin's lymphomas, kidney cancer, glioblastoma, and breast cancer. High levels of PD-L1 are often found in these, as well as some other types of cancer and can be treated effectively with immunotherapy using the inventive antibodies.

The invention provides therapeutic methods in which the antibody or antibody fragment of the invention is useful to treat above mentioned cancers. The antibodies or antigen-binding fragments of the invention are particularly useful for combination therapies for example with histone deacetylase (HDAC) inhibitors (HDIs). HDIs have a long history of use in psychiatry and neurology as mood stabilizers and anti-epileptics. More recently they are being investigated as possible treatments for cancers and inflammatory diseases.

The "classical" HDIs act exclusively on Class I, II and Class IV HDACs by binding to the zinc-containing catalytic domain of the HDACs. These classical HDIs can be classified into several groupings named according to the chemical moiety that binds to the zinc ion (except cyclic tetrapeptides which bind to the zinc ion with a thiol group). Some examples in decreasing order of the typical zinc binding affinity:
1. hydroxamic acids (or hydroxamates), such as trichostatin A,
2. cyclic tetrapeptides (such as trapoxin B), and the depsipeptides,
3. benzamides,
4. electrophilic ketones, and
5. the aliphatic acid compounds such as phenylbutyrate and valproic acid.

"Second-generation" HDIs include the hydroxamic acids vorinostat (SAHA), belinostat (PXD101), LAQ824, and panobinostat (LBH589); and the benzamides : entinostat (MS-275), tacedinaline (CI994), and mocetinostat (MGCD0103).

The sirtuin Class III HDACs are dependent on NAD+ and are, therefore, inhibited by nicotinamide, as well as derivatives of NAD, dihydrocoumarin, naphthopyranone, and 2-hydroxynaphthaldehydes.

The antibodies or antigen-binding fragments of the invention are particularly useful for combination therapies with one or more of any agent selected from the group consisting of: chemotherapy drugs and/or cytostatic drugs of any kind, such as for example alkylating agents, antimetabolites, anthracyclines, taxanes, vinca alkaloids. Specific substances used in such a treatment may be for example cyclophosphamide, methotrexate, 5-fluoruracil, vincristin, prednisolone, epirubicin, cisplatin, folinic acid, irinotecan, oxaliplatin, mitomycin C, etoposid, bleomycin, procarbazin, lomustin, adriamycin, and/or capecitabin.

In one embodiment entinostat, also known as SNDX-275 and MS-275, a benzamide histone deacetylase inhibitor undergoing clinical trials for treatment of various cancers, is used.

In one embodiment the inventive bispecific monoclonal antibody or antigen-binding fragment or a substantially similar variant thereof can be used to from an antibody-drug-conjugate (ADC). Antibody-drug conjugates or ADCs are a class of biopharmaceutical drugs designed as a targeted therapy for treating cancer. Unlike chemotherapy, ADCs are intended to target and kill tumor cells while sparing healthy cells.

ADCs combine the targeting capabilities of monoclonal antibodies with the cancer-killing ability of cytotoxic drugs. They can be designed to discriminate between healthy and diseased tissue.

In one embodiment an anti-cancer drug is coupled to the inventive antibodies that specifically targets tumors displaying EGFR and PD-L1 on their surface. The biochemical reaction between the antibody and the target protein (antigen) triggers a signal in the tumor cell, which then absorbs or internalizes the antibody together with the linked cytotoxin. After the ADC is internalized, the cytotoxin kills the cancer. This targeting limits side effects and gives a wider therapeutic window than other chemotherapeutic agents.

The drug may be a cytotoxic payload selected from the group consisting of mertansine (DM1), duocarmycin (DUO), auristatin F-hydroxypropylamide (AF-HPA), monomethylauristatin F (MMAF), pyrrolobenzodiazepines (PBDs), monomethyl auristatin E (MMAE), calicheamicin (calich.), ravtansine (DM4), active metabolite of irinotecan (SN-38), novel cytotoxic dolastatin 10 analogue (PF06380101), DX-8951 a derivative of the camptothecin analog exatecan (DXd), doxorubicin (DOX), and auristatin-0101 (Aur0101) and/or any combination thereof.

The antibody may be connected to the drug directly or via a linker. A stable ADC linker ensures that less of the cytotoxic payload falls off before reaching a tumor cell, improving safety, and limiting dosages.

Linkers are based on chemical motifs including disulfides, hydrazones or peptides (cleavable), or thioethers (noncleavable). Cleavable and noncleavable linkers were proved to be safe in preclinical and clinical trials. For example "brentuximab vedotin" includes an enzyme-sensitive cleavable linker that delivers the antimicrotubule agent monomethyl auristatin E or MMAE, a synthetic antineoplastic agent, to human-specific CD30-positive malignant cells. MMAE inhibits cell division by blocking the polymerization of tubulin. Because of its high toxicity MMAE cannot be used as a single-agent chemotherapeutic drug. However, MMAE linked to the antibodies of the invention may be used for a specific tumor-cell targeted approach.

Another example "trastuzumab emtansine" is a combination of the microtubule-formation inhibitor mertansine (DM-1) and antibody trastuzumab that employs a stable, non-cleavable linker.

The availability of better and more stable linkers has changed the function of the chemical bond. The type of linker, cleavable or noncleavable, lends specific properties to the cytotoxic drug. For example, a non-cleavable linker keeps the drug within the cell. As a result, the entire antibody, linker and cytotoxic (anti-cancer) agent enter the targeted cancer cell where the antibody is degraded into an amino acid. The resulting complex - amino acid, linker and cytotoxic agent - is considered to be the active drug. In contrast, cleavable linkers are detached by enzymes in the cancer cell. The cytotoxic payload can then escape from the targeted cell and, in a process called "bystander killing", attack neighbouring cells.

Another type of cleavable linker, currently in development, adds an extra molecule between the cytotoxin and the cleavage site. This allows researchers to create ADCs with more flexibility without changing cleavage kinetics. Researchers are developing a new method of peptide cleavage based on Edman degradation, a method of sequencing amino acids in a peptide. Also under development are site-specific conjugation (TDCs) and novel conjugation techniques to further improve stability and therapeutic index, α emitting immunoconjugates, antibody-conjugated nanoparticles and antibody-oligonucleotide conjugates.

In another aspect of the present invention the inventive antibodies may be combined with further binding sites for other targets in order to increase the specificity of the antibody even further. This is possible due to the specific nature of the antibodies presented therein which can bind to EGFR / PD-L1 with just one Fab-region and/or one antibody-arm only. In case of scFv-fragments one could imagine the fusion to a second or further scFv-fragment able to bind at least one other target, creating trivalent, tetravalent or multivalent constructs. Obviously also other combinations with other antigen-binding proteins could be envisaged, for example scFv-fragment-ligand combination, scFv-fragment-anticaline-combination and many more (for example other small modular immunopharmaceuticals (SMIPs)).

In another aspect of the present invention the inventive antibodies are used in chimeric antigen receptor-therapy (CAR-T-cell-therapy). The inventive antibodies may serve as one of the chimeric antigen-receptors, or may be used in combination in order to improve the specificity of the modified T-cells.

In another aspect of the present invention the inventive antibodies may be used as sensors in a device or binding entity in an assay, e.g. an ELISA in order to stratify patients and/or patient groups before, during and/or after undergoing therapy.

### Short Description of the Figures

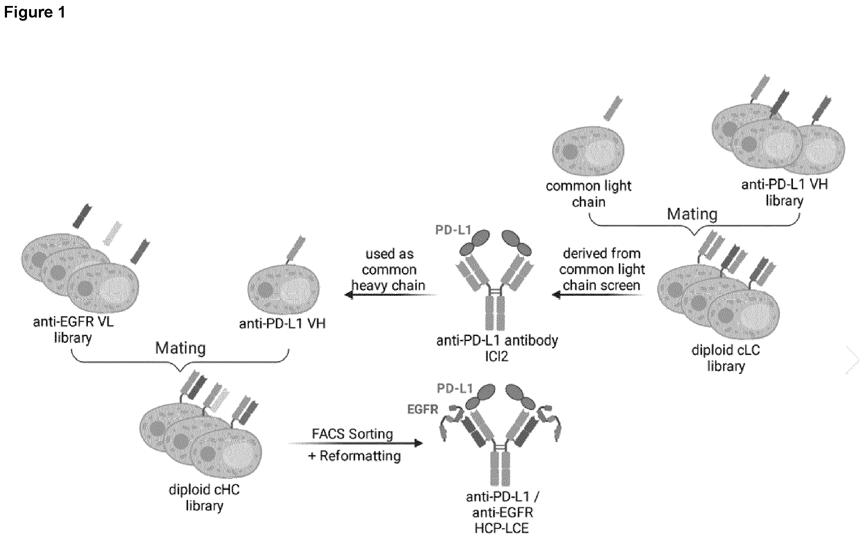

**Figure 1****: Schematic representation demonstrating the generation of the Two-in-One antibody HCP-LCE.**
   The anti-PD-L1 antibody ICI2 is derived from a common light chain YSD library (SEQ ID No.: 16). The VH fragment of the anti-PD-L1 antibody ICI2 was paired with an anti-EGFR VL library by yeast mating. FACS screening and subsequent reformatting into the full-length antibody format enabled the isolation and production of a Two-in-One antibody targeting PD-L1 and EGFR. Created with BioRender.com.

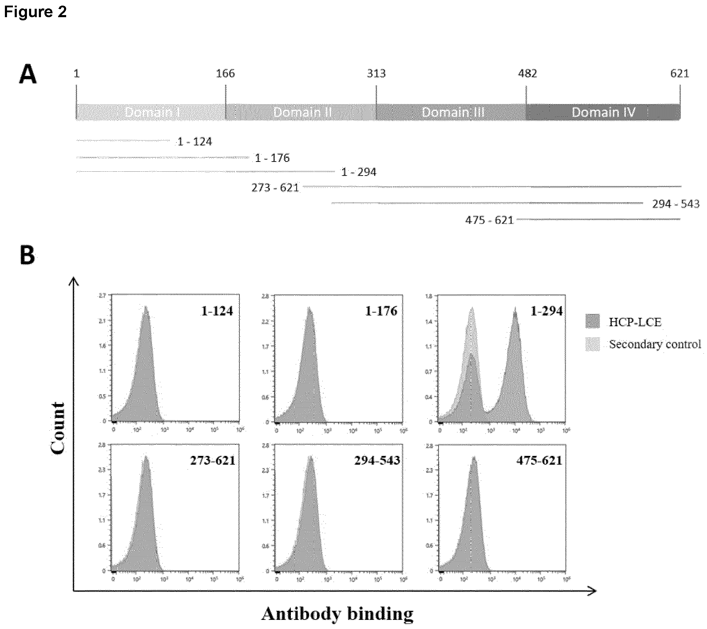

**Figure 2****: YSD-based EGFR epitope mapping of HCP-LCE.**
   **(A)** Schematic representation of EGFR extracellular domains I to IV and the six EGFR fragments investigated in this study. **(B)** Binding of HCP-LCE to yeast cells expressing different truncated EGFR fragments was detected using the goat anti-human Fc PE antibody. Measurements without HCP-LCE served as secondary control. HCP-LCE binds to EGFR fragment 1-294.

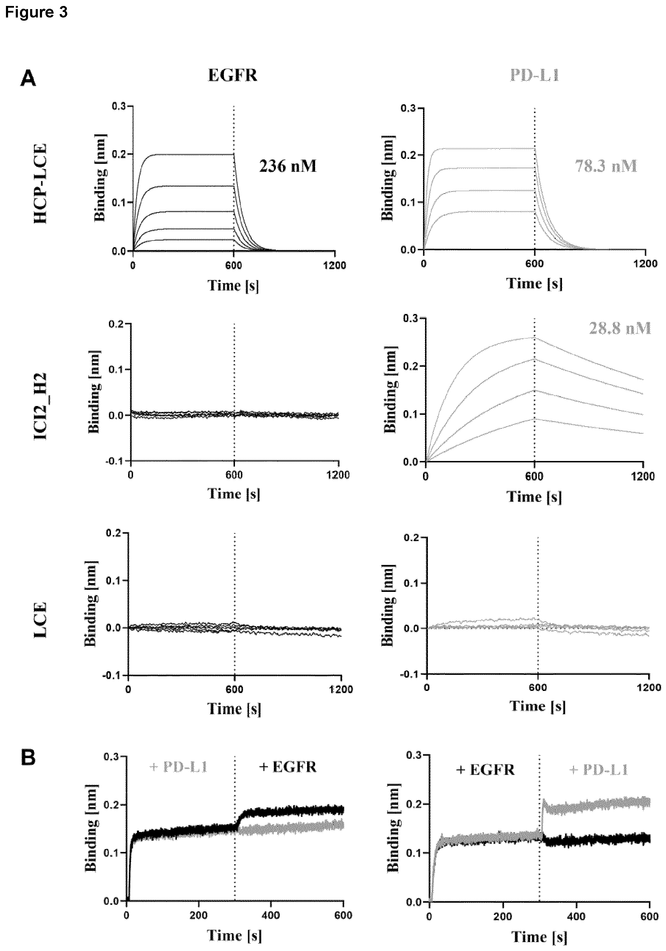

**Figure 3****: Characterization of antigen binding of the Two-in-One antibody HCP-LCE by BLI-measurements.**
   **(A)** BLI-measurements of HCP-LCE, ICI2_H2 and LCE against EGFR and PD-L1. HCP-LCE binds both antigens, whereas ICI2_H2 exclusively targets PD-L1. LCE shows no binding to either antigen. **(B)** BLI-assisted simultaneous binding assay. The one-armed HCP-LCE variant was loaded onto biosensors and antigens are added step-wise, revealing simultaneous EGFR and PD-L1 binding.
**Figure 4****: EGF competition and EGFR signalling assays.**
   (A) BLI-assisted EGF competition assay. HCP-LCE was loaded onto AHC biosensors and subsequently associated to EGFR pre-incubated with varying EGF concentrations. HCP-LCE binds to EGFR despite EGF binding. (B) Cell-based EGFR signalling assay. EGF-induced AKT phosphorylation was analysed in EGFR-positive A549 cells. SEB7, ICI2_H2, the bispecific construct SEB7xICI2_H2, cetuximab and durvalumab were tested in comparison to the Two-in-One antibody HCP-LCE. All measurements were performed in triplicates.
**Figure 5****: PD-1 competition and PD-1/PD-L1 blockage assays.**
   (A) BLI-assisted PD-1 competition assay. HCP-LCE was loaded onto FAB2G-biosensors and subsequently associated to PD-L1 pre-incubated with varying PD-1 concentrations. The binding of HCP-LCE to PD-L1 at different PD-1 concentrations reveals dose-dependent binding. (B) Cell-based PD-1/PD-L1 blockage assay. SEB7, ICI2_H2, the bispecific construct SEB7xICI2_H2 and durvalumab were tested in comparison to the Two-in-One antibody HCP-LCE. EC₅₀ values: durvalumab, 0.39 nM; ICI2_H2, 8.60 nM; SEB7xICI2_H2, 179 nM; HCP-LCE, 214 nM. Luciferase activity is plotted against the logarithmic antibody concentration. All measurements were performed in duplicates, and the experiments were repeated at least three times, yielding similar results.

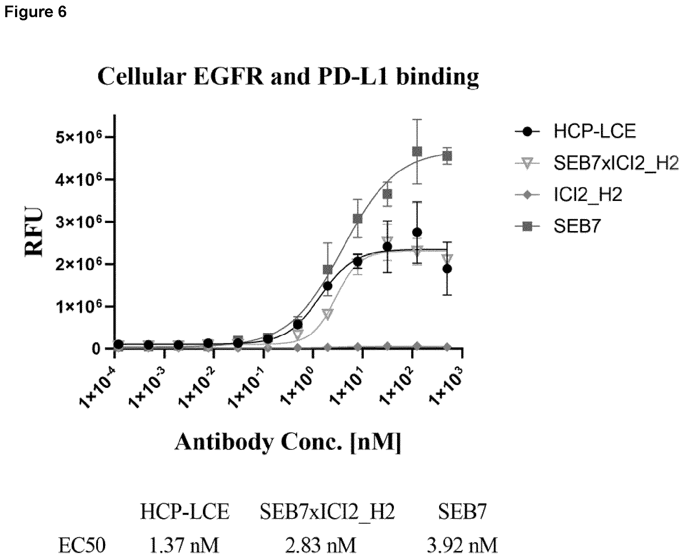

**Figure 6****: Cell binding of EGFR and PD-L1 on A431 cells.**
   Cell titration of HCP-LCE, ICI2_H2, SEB7 and the bispecific construct on EGFR/PD-L1 double positive A431 cells. A variable slope four-parameter fit was utilized to fit the resulting curves. EC₅₀ values: HCP-LCE, 1.37 nM; SEB7, 3.92 nM; SEB7xICI2_H2, 2.83 nM. All measurements were performed in triplicates, and the experiments were repeated at least three times, yielding similar results.
**Figure 7****: Trispecific variants based on the Two-in-One antibody HCP-LCE.**
   A schematic representation of the structure and arrangement of the trispecific antibody variants is depicted, as well as their corresponding one-armed variants.
**Figure 8****: BLI-assisted simultaneous binding assay..**
   The Knob-into-hole antibody or the one-armed variants were loaded onto biosensors and antigens are added step-wise, revealing simultaneous PD-L1, EGFR and CD16 binding.

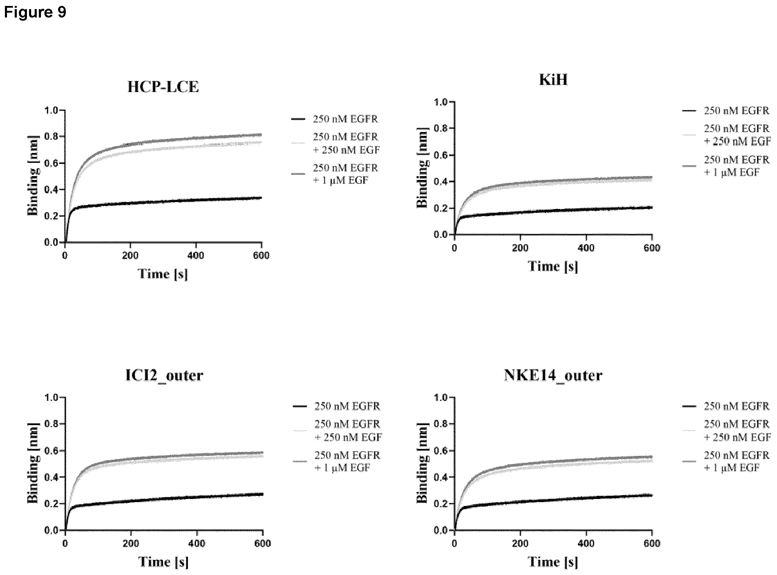

**Figure 9****: BLI-assisted EGF competition assay.**
   The trispecific antibodies based on the Two-in-One antibody HCP-LCE were loaded onto AHC biosensors and subsequently associated to EGFR pre-incubated with varying EGF concentrations. The binding of the antibodies to EGFR at different EGF concentrations reveals dose-dependent binding.

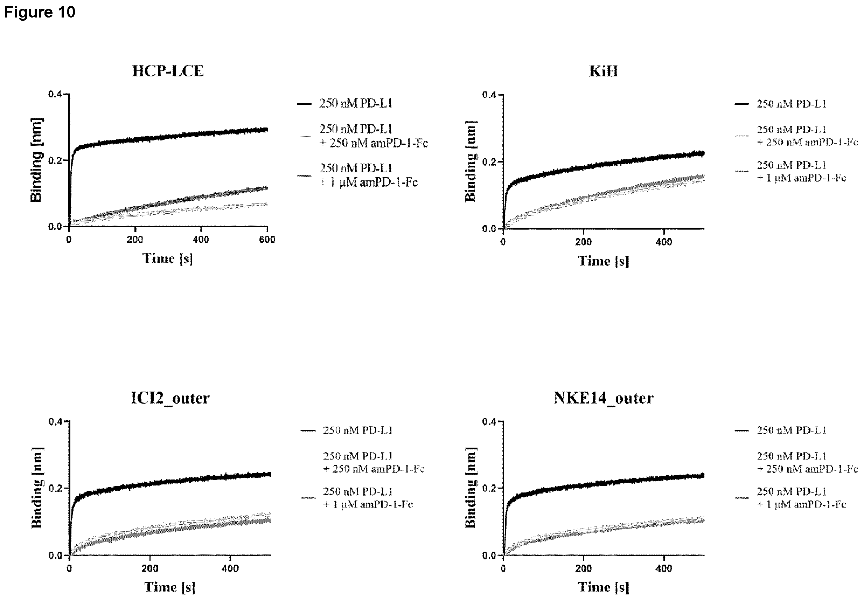

**Figure 10****: BLI-assisted PD-1 competition assay.**
   The trispecific antibodies based on the Two-in-One antibody HCP-LCE were loaded onto FAB2G-biosensors and subsequently associated to PD-L1 pre-incubated with varying PD-1 concentrations. The binding of the antibodies to PD-L1 at different PD-1 concentrations reveals dose-dependent binding.

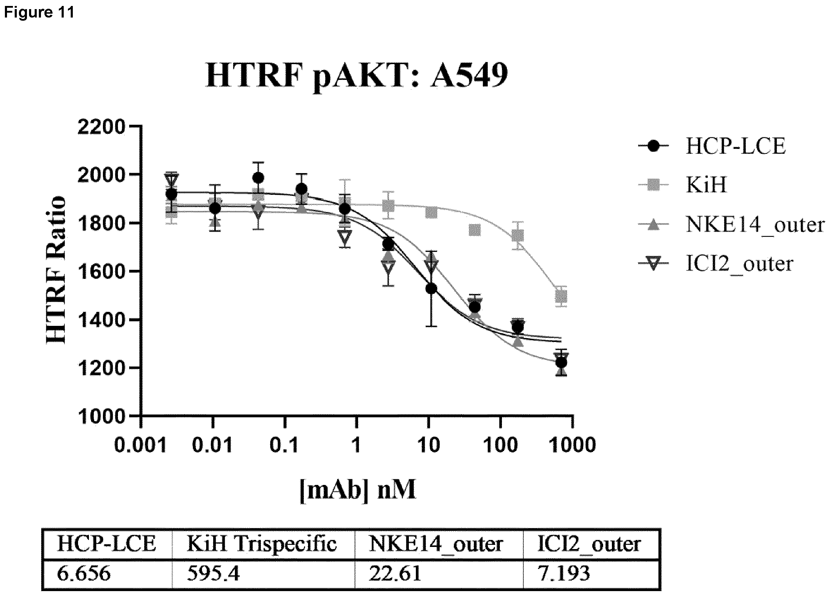

**Figure 11****: EGFR signaling inhibition.**
   The antibodies were tested for their capability to inhibit phospho-AKT using the A549 cell line. HTRF Ratio is shown on the y-axis, the antibody concentration is shown on the x-axis.
**Figure 12****: Affinity measurements.**
   Binding kinetics of SEB7, SEB7xICI2_H2 and oaHCP-LCE to EGFR and PD-L1.
**Figure 13****: SDS-PAGE analysis.**
   SDS-PAGE analysis was done with ICI2_H2, SEB7, HCP-LCE, SEB7xICI2_H2, oaHCP-LCE, LCE, KiH, ICI2_outer and NKE14_outer under reducing conditions revealing high purity and the expected molecular weights. SDS-PAGE analysis of KiH, ICI2_outer, NKE14_outer and HCP-LCE under non-reducing conditions revealing the expected molecular weights.

### Sequences

In this application the following sequences are used:
SEQ ID No. 1: Inventive antibody (HCP-LCE), full variable heavy chain amino acid sequence.
SEQ ID No. 2: Inventive antibody (HCP -LCE), full variable light chain amino acid sequence.
SEQ ID No. 3: Inventive antibody (HCP -LCE), CDR-H1 amino acid sequence.
SEQ ID No. 4: Inventive antibody (HCP -LCE), CDR-H2 amino acid sequence.
SEQ ID No. 5: Inventive antibody (HCP -LCE), CDR-H3 amino acid sequence.
SEQ ID No. 6: Inventive antibody (HCP -LCE), CDR-L1 amino acid sequence.
SEQ ID No. 7: Inventive antibody (HCP -LCE), CDR-L2 amino acid sequence.
SEQ ID No. 8: Inventive antibody (HCP -LCE), CDR-L3 amino acid sequence.
SEQ ID No. 9: CD16-binding antibody (NKE14), full variable heavy chain amino acid sequence.
SEQ ID No. 10: CD16-binding antibody (NKE14), CDR-H1 amino acid sequence.
SEQ ID No. 11: CD16-binding antibody (NKE14), CDR-H2 amino acid sequence.
SEQ ID No. 12: CD16-binding antibody (NKE14), CDR-H3 amino acid sequence.
SEQ ID No. 13: Inventive antibody (HCP -LCE), full length amino acid sequence of the heavy chain.
SEQ ID No. 14: Inventive antibody (HCP -LCE), full length amino acid sequence of the light chain.
SEQ ID No. 15: Common variable heavy chain used in yeast display, amino acid sequence of the heavy chain.
SEQ ID No. 16: Common variable light chain used in yeast display, amino acid sequence of the light chain.
SEQ ID No. 17: Knob-into-hole (KiH) variant, amino acid sequence of the heavy chain, "knob"-version.
SEQ ID No. 18: Knob-into-hole (KiH) variant, amino acid sequence of the heavy chain, "hole"-version.
SEQ ID No. 19: Knob-into-hole (KiH) variant, amino acid sequence of the light chain.
SEQ ID No. 20: ICI2_outer & NKE14_inner variant, amino acid sequence of the heavy chain (full length).
SEQ ID No. 21: ICI2_outer & NKE14_inner variant, amino acid sequence of the light chain (full length).
SEQ ID No. 22: NKE14_outer & ICI2_inner variant, amino acid sequence of the heavy chain (full length).
SEQ ID No. 23: NKE14_outer & ICI2_inner variant, amino acid sequence of the light chain (full length).
SEQ ID No. 24: The one-armed variants (oa) all used the same light chain amino acid sequence (full length) depicted here.
SEQ ID No. 25: The one-armed variants (oa) all used the same heavy chain ("hole"-version) amino acid sequence (full length) depicted here.
SEQ ID No. 26: The one-armed variants (oa) ICI2_outer & NKE14_inner variant used the heavy chain ("knob"-version) amino acid sequence (full length) depicted here.
SEQ ID No. 27: The one-armed variants (oa) NKE14_outer & ICI2_inner variant used the heavy chain ("knob"-version) amino acid sequence (full length) depicted here.

Further proteins are used hereinunder, which sequence and further information is available from the UniProt-database (https://www.uniprot.org/) (version of February 2, 2021):
CD16: UniProtKB - P08637
PD-1: UniProtKB - Q15116
PD-L1: UniProtKB - Q9NZQ7
EGF: UniProtKB - P01133
EGFR: UniProtKB - P00533

Furthermore, the antibody durvalumab (MEDI4736) is a commercially available antibody, which has been published for example in the National Library of Medicine (https://pubchem.ncbi.nlm.nih.gov/substance/249565666) (Version 11, September 2, 2021).

The antibody cetuximab is a commercially available antibody, which has been published in the Kegg-library (https://www.genome.ip/entry/D03455) (Version February 28, 2022).

The antibody SEB7 has been published by Bogen et al., Front. Immunol., 23 December 2020, https://doi.org/10.3389/fimmu.2020.606878.

### Examples

### Example 1: Chicken Library Generation and Screening for ICI2

Based on the spleen-derived RNA from a chicken (*Gallus gallus*) immunized with four doses of commercially available PD-L1-Fc, cDNA was synthesised and VH genes were amplified by PCR. VH-encoding PCR amplicons were subcloned into a pYD1-derived vector and subsequently transformed into EBY100 yeast cells. BJ5464 yeast cells harbouring a pYD1-derived vector encoding a common light chain were combined with these EBY100 cells by yeast mating. The resulting diploid library was sorted by FACS for two rounds for Fab display and binding to decreasing concentrations of PD-L1. In the third round, a competition staining with PD-L1 and PD-1 was performed in order to isolate binders against the interaction site of PD-L1 and PD-1. The sort outcome of the third round only exhibited antibodies binding PD-L1, in one embodiment with an overlapping epitope with durvalumab. All unique binders derived from this campaign were produced as mAbs. Subsequent BLI analysis revealed that ICI2 exhibits the highest affinity towards PD-L1 and was therefore chosen for further development.

### Example 2: Two-in-One Library Generation and Screening

In order to generate a Two-in-One antibody, we sought to combine the heavy chain of a chicken-derived antibody with a chicken-derived immune YSD light chain library followed by subsequent screening for binding properties towards two antigens. As model targets, the extracellular domains of PD-L1 (PD-L1-ECD) and EGFR (EGFR-ECD) were chosen. Currently, various monoclonal antibodies targeting either EGFR, including the therapeutic antibodies panitumumab, necitumumab, nimotuzumab and cetuximab, or PD-L1, among them durvalumab, avelumab and atezolizumab are approved for tumor treatment in multiple countries. Recently, our group isolated a chicken-derived anti-PD-L1 antibody called ICI2. Since ICI2 exhibited a common light chain as it was utilized in a multispecific setup, we assumed that the heavy chain CDRs are mainly responsible for antigen recognition and could tolerate various light chains, as reported for other antibodies. In order to isolate a Two-in-One antibody targeting both PD-L1 and EGFR, the ICI2 heavy chain was paired with a diversity of anti-EGFR light chains (Figure 1). The light chain library was generated by amplification of VL genes from cDNA derived from a chicken immunized with EGFR-ECD and subsequent insertion into a pYD1-derived vector encoding a human lambda CL by homologous recombination in BJ5464 yeast cells. The light chain diversity of 2.9×10⁸ transformants was combined with EBY100 yeast cells encoding the ICI2 VH-CH1 fragment by yeast mating (Figure 1), resulting in adequate oversampling of the estimated light chain diversity, which is estimated to be about 5×10⁸ unique variants.

This diploid common heavy chain yeast library was screened by FACS over three consecutive sorting rounds with 250 nM EGFR-Fc. This yielded in the enrichment of a yeast population carrying the genes for Fab fragments recognizing both EGFR-Fc and PD-L1-Fc with 250 nM of the respective antigen. Fc binding could be excluded based on binding analysis to an unrelated Fc fusion protein. Sequence analysis of ten randomly chosen clones revealed one distinct VL sequence, which was enriched in the sorting process.

### Example 3: EGFR Epitope Mapping on the subdomain level

Expi293F cells were co-transformed using the isolated VL sequence reformatted into a pTT5-derived vector encoding a lambda CL sequence and a pTT5 vector encoding the ICI2 heavy chain. Purification of the Two-in-One antibody HCP-LCE (heavy chain PD-L1 - light chain EGFR), was performed using Protein A affinity chromatography.

The extracellular region of EGFR consists of two homologous ligand-binding domains (domains I and III) and two cystine-rich domains (domains II and IV). The binding of EGF to the EGFR monomers at domains I and III promotes domain rearrangement to expose the dimerization arm in domain II finally resulting in the generation of EGFR homodimers. For full EGFR activation, ligand binding and EGFR dimerization are crucial.

To analyze which of the four extracellular EGFR domains is targeted by HCP-LCE, flow cytometric analysis was performed using yeast cells displaying truncated fragments of the EGFR-ECD. Since HCP-LCE exclusively targets EGFR fragment 1-294 but neither 1-124 nor 1-176 (Figure 2), it was mapped to EGFR domain II, which is involved in receptor dimerization. Cetuximab is known to target domain III inhibiting EGF-binding to EGFR, while matuzumab blocks the receptor activation by sterically preventing the domain rearrangement. Binding of HCP-LCE to EGFR domain II may suggest inhibition of receptor dimerization and subsequent activation.

### Example 4: Affinity measurement

BLI measurements were performed in order to determine the affinity of HCP-LCE to PD-L1 and EGFR (cf. Figure 3). In addition, the affinity of a one-armed HCP-LCE variant (oaHCP-LCE) was determined. Therefore the Knob-into-Hole technology was utilized to ensure heterodimerization of the heavy chains of oaHCP-LCE. A His-Tag C-terminally fused to the Hole-Fc and a TwinStrep-Tag C-terminally cloned to the Knob-Fc allowed two-step purification by immobilized metal affinity chromatography (IMAC), followed by StrepTactin XT chromatography. Furthermore, affinity measurements of the HCP-LCE heavy chain combined with an unrelated light chain (ICI2_H2) and that of the HCP-LCE light chain together with an unrelated heavy chain (LCE) to the two proteins of interest were performed.

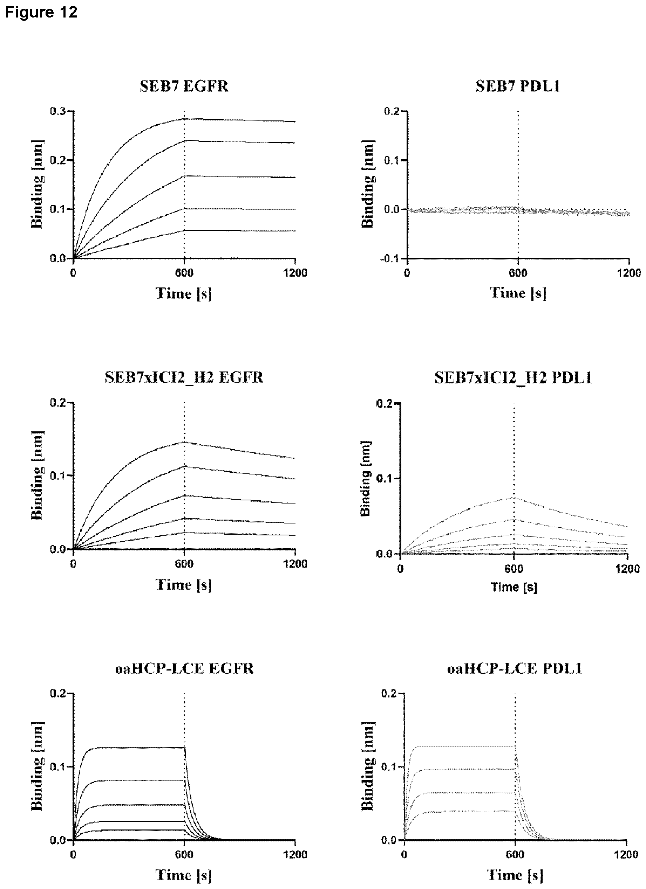

HCP-LCE was able to bind both antigens with a KD of 78.3 nM and 236 nM for PD-L1 and EGFR binding, respectively, exhibiting a high off-rate (Figure 3A, as well as Table 2 in example 8 below). The one-armed variant showed slightly lower affinities which might presumably be caused by the lower avidity (Figure 12). Variant ICI2_H2 exclusively targeted PD-L1 with affinity in the double digit nanomolar range, whereas variant LCE showed no binding to either PD-L1 or EGFR (Figure 3A, as well as Table 2 in example 8 below). This suggests that only the three HCP-LCE heavy chain CDRs are responsible for PD-L1 binding, contrary to EGFR binding involving overlapping heavy and light chain CDRs.

Since EGFR and PD-L1 are widely expressed on healthy cells, simultaneous binding of both proteins is essential to increase tumor-specificity. To analyze the binding behaviour of HCP-LCE and to verify whether both antigens can be targeted simultaneously with a single Fab, oaHCP-LCE was loaded onto AHC biosensors and sequentially incubated with both target proteins of interest. It is essential to use the one-armed variant, since the symmetric HCP-LCE antibody could target one antigen with each Fab fragment. Binding to PD-L1 first and EGFR second as well as reverse binding was considered. The oaHCP-LCE variant was able to bind both antigens simultaneously, regardless of the order of target protein incubation (Figure 3B). These findings indicate that the paratopes do not overlap.

### Example 5: Biophysical Characterization

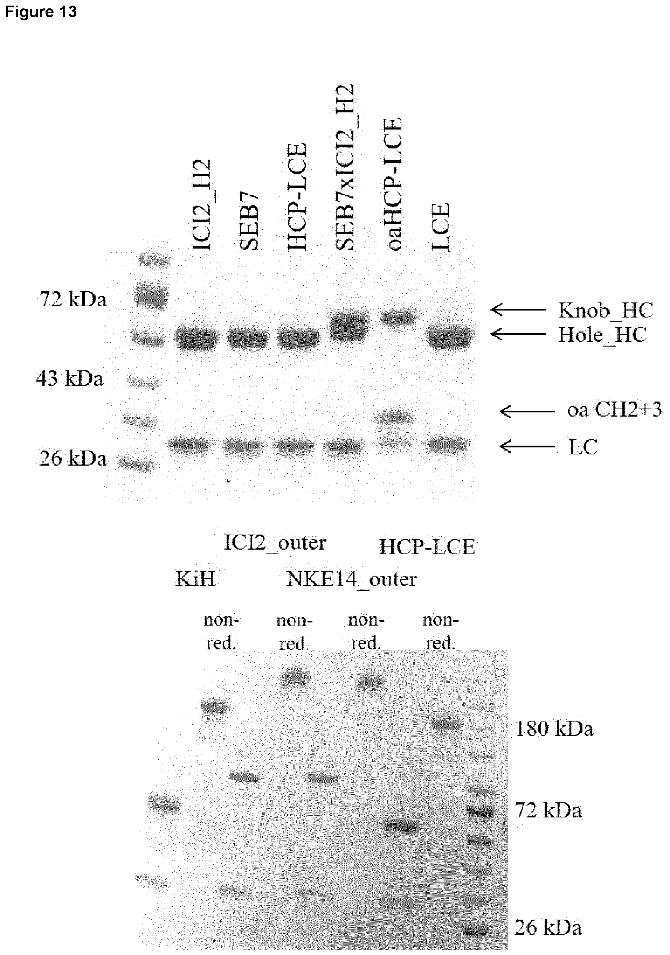

Size-exclusion chromatography (SEC) profiles demonstrated that HCP-LCE exhibited favorable properties with almost no measurable aggregation (Table 2). Monospecific antibodies ICI2_H2 and LCE showed an excellent aggregation profile, while the bispecific EGFR- and PD-L1-binding antibody SEB7xICI2_H2 and the oaHCP-LCE variant exhibited aggregation of 10.8% and 11.20%, respectively (Table 2). Retention times were as expected (Table 2), indicating the accurate size of the antibodies produced, which is in accordance with SDS-PAGE analysis (Figure 13). In the case of oaHCP-LCE and SEB7xICI2_H2, SDS-PAGE demonstrated the expression of similar amounts of both heavy chains, with the TwinStrep-tagged Fc having a significantly higher molecular weight than the His-tagged Fc (Figure 13).

Thermal stability was analyzed using NanoDSF, yielding T_{M} values between 58.9°C and 67.3°C, indicating high thermal stability of all variants (Table 2). For full-length antibodies, two to three T_{M} values are expected based on unfolding of the Fab fragment and CH2/CH3 domains. The lowest T_{M} value was utilized to compare the stability of all antibodies generated.

### Example 6: EGF and PD-1 Competition

To investigate antibody-mediated ligand receptor blockade, a competition assay was performed by aid of biolayer interferometry (cf. Figures 4 & 5). For analysis of EGF competition, Anti-human IgG Fc Capture (AHC) biosensors were loaded with HCP-LCE and subsequently associated to 250 nM EGFR pre-incubated with 250 nM or 1000 nM EGF. Due to the binding of EGFR to EGF, the complex exhibits a larger molecular size compared to EGFR alone. Binding of HCP-LCE to this complex, therefore, results in a higher increase in layer thickness compared to binding of EGFR alone (Figure 4A), indicating that the antibody does not target the interaction site of EGF and EGFR, which is consistent with the YSD-based epitope mapping experiment (Figure 2B). EGF binds simultaneously to EGFR domains I and III, whereas HCP-LCE targets EGFR domain II, which is involved in receptor dimerization.

For total EGFR activation, ligand binding and EGFR dimerization are essential. EGF binding to EGFR promotes domain rearrangement to expose the dimerization arm in domain II. Since HCP-LCE targets domain II, it was investigated whether the Two-in-One antibody is able to inhibit EGF-induced EGFR dimerization by measuring the downstream phosphorylation of AKT in EGFR-positive A549 cells. In the presence of HCP-LCE (100µg/mL), AKT phosphorylation is significantly reduced compared to the EGF-stimulated control (20 ng/mL) (Figure 4B). Anti-EGFR antibody SEB7 and SEB7xICI2_H2, which also target EGFR domain II, showed comparable inhibition of AKT phosphorylation as HCP-LCE. The EGFR domain III binder Cetuximab completely inhibited EGF-induced phosphorylation of AKT (Figure 4B) since binding of domain III blocked EGF binding. To conclude, EGFR signalling is significantly inhibited by HCP-LCE binding to dimerization domain II without interfering EGF-binding to its receptor.

To investigate HCP-LCE-mediated PD-1/PD-L1 competition, HCP-LCE was loaded onto FAB2G biosensors and was associated to 250 nM PD-L1 pre-incubated with either 250 nM or 1000 nM of PD-1 (PD-1). HCP-LCE exhibited significantly impaired binding to PD-L1 in the presence of PD-1, indicating that the antibody targets and blocks the PD-1/PD-L1 interaction site (Figure 5A).

Verification of the PD-L1 blockage activity of HCP-LCE was performed in a cell-based context using the Promega^{®} PD-L1 blockade assay kit. HCP-LCE showed notable PD-L1 blockade, although with a less dominant effect compared with ICI2_H2 (Figure 5B). This diminished EC₅₀ value most probably originates from the lower affinity PD-L1 binding. However, in combination with the original common light chain dFEB4-1, the ICI2 heavy chain exhibited a blockage of the PD-1/PD-L1 interaction comparable to the commercially available antibody durvalumab, as well as a significantly higher affinity PD-L1 binding. SEB7xICI2_H2 exhibited comparable PD-L1 blockade as HCP-LCE, despite monovalent target binding of the bispecific antibody. The anti-EGFR antibody SEB7 did not interfere with PD-1/PD-L1 interaction even at high concentrations (Figure 5B). Conclusively, the binding of PD-1 to PD-L1 is significantly inhibited by HCP-LCE, indicating its function as a checkpoint inhibitor.

### Example 7: Cell Titration on A431 Cells

Since HCP-LCE is expected to provide increased tumor-specificity compared to the bispecific antibody SEB7xICI2_H2 and the corresponding monospecific antibodies due to simultaneous binding of EGFR and PD-L1 at each Fab arm, cell binding experiments were performed on EGFR/PD-L1 double-positive A431 cells by flow cytometry. Cells were stained with the respective antibody at a concentration ranging from 0.12 pM to 500 nM utilizing a four-fold dilution series and binding was verified using an anti-human Fc PE detection antibody. HCP-LCE exhibited cellular binding with an EC₅₀ value of 1.37 nM, while the EC₅₀ value of the bsAb SEB7xICI2_H2 was about two times higher (Figure 6). EGFR expression on cancer cells typically strongly exceeds that of PD-L1, as demonstrated by A431 binding of the monospecific antibodies (Figure 6). The antibodies analyzed did not show binding to EGFR/PD-L1 double-negative HEK cells, excluding non-specific cell binding. These data indicate that simultaneous binding of EGFR and PD-L1 at both Fab arms enhances A431 cell binding, compared to monovalent or bivalent target protein binding.

### Example 8: Multispecific variants

Based on the Two-in-One antibody HCP-LCE (cf. Figure 7, "HCP-LCE"), five different trispecific variants were designed (cf. Figure 7, "V1" - "V5"). All variants are based on the LCE as common light chain.
1. A "Knob-into-Hole" (KiH) variant (cf. Figure 7, "V1") consisting of HCP-LCE on one side and the heavy chain of the CD16 binder, paired with the HCP-LCE light chain (SEQ ID No.: 17, 18, 19).
2. Two variants with two Fabs (see KiH variant) each side in both possible orientations (cf. Figure 7, "V2" and "V4"):
   - ICI2_outer and NKE14_inner (SEQ ID No.: 20/21)
   - NKE14_outer and ICI2_inner (SEQ ID No.: 22/23)
3. both variants were also designed and produced as one-armed (oa) variants (cf. Figure 7, "V3" and "V5") (SEQ ID No.: 24, 25, 26, 27).

Purification of antibodies V2 (ICI2_outer) and V4 (NKE14_outer) was performed via Protein A chromatography. For the KiH variant (V1) and the oa variants (V3 and V5) a His-Tag was C-terminally fused to the Hole-Fc and a Twin-Strep-Tag C-terminally cloned to the Knob-Fc. This allowed two-step purification by IMAC, followed by StrepTactin XT chromatography.

The accurate size of the antibodies is in accordance with SDS-PAGE analysis (Figure 13).

BLI measurements were performed in order to determine the affinity of the trispecific variants to PD-L1, EGFR and CD16. All variants were able to bind the antigens with KD values shown in the table below.

**Table 1: BLI-results for different antibody-variants of the present invention**

| **Variant** | **Antibody** | **EGFR** | **PD-L1** | **CD16** |
|---|---|---|---|---|
| V1 | KiH | 570 nM | 104 nM | 277 nM |
| V2 | ICI2_outer Fab | 460 nM | 97.6 nM | 382 nM |
| V3 | oalCI2_outer | 763 nM | 115 nM | 346 nM |
| V4 | NKE14_outer Fab | 603 nM | 125 nM | 302 nM |
| V5 | oaNKE14_outer | 982 nM | 137 nM | 342 nM |
| Comparative mAb | NKE14+LCE | - | - | 151 nM |
| "HCP-LCE" | HCP-LCE | 236 nM | 78.3 nM | - |
| one arm version of HCP-LCE | oaHCP-LCE | 294 nM | 79.5 nM | - |

To analyze whether all three antigens can be targeted simultaneously, the one-armed variants were loaded onto AHC biosensors and sequentially incubated with the three proteins of interest. Binding to PD-L1 first, EGFR second and CD16 third was considered.

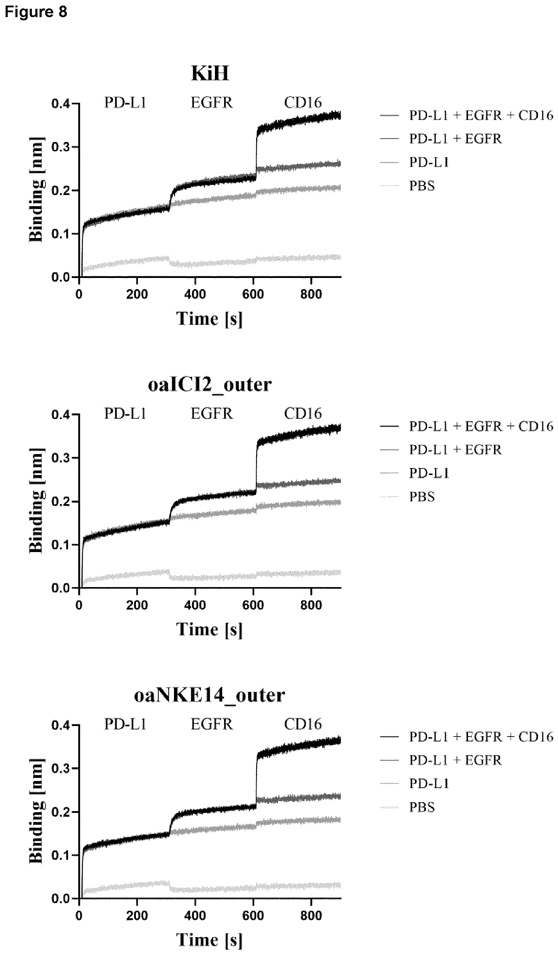

All variants were able to bind the three antigens simultaneously, shown in Figure 8.

### Example 9: Biophysical properties of different antibodies.

**Table 2: Biophysical properties of HCP-LCE, oaHCP-LCE, LCE, ICI2_H2 and SEB7xICI2_H2 including affinity, kinetic binding rates, melting temperature and aggregation.**

| **Antibody** | **K_{D} [nM]** | | **kₒₙ [M⁻¹ s⁻¹]** | | **k_{dis} [s⁻¹]** | | **T_{M} [°C]** | **Aggreg ation [%]** |
|---|---|---|---|---|---|---|---|---|
| | **EGFR** | **PD-L1** | **EGFR** | **PD-L1** | **EGFR** | **PD-L1** | | |
| HCP-LCE | 236 ± 10.7 | 78.3 ± 1.36 | 4.15 × 10⁵ ± 1.58 × 10⁴ | 9.73 × 10⁵ ± 1.37 × 10⁴ | 9.81 × 10⁻² ± 2.41 × 10⁻³ | 7.62 × 10⁻² ± 7.78 × 10⁻⁴ | 58.9 | 2.22 |
| oaHCP-LCE | 295 ± 17.1 | 117 ± 4.15 | 3.88 × 10⁵ ± 1.92 × 10⁴ | 9.29 × 10⁵ ± 2.62 × 10⁴ | 1.14 × 10⁻¹ ± 3.52 × 10⁻³ | 1.09 × 10⁻¹ ± 2.32 × 10⁻³ | 59.6 | 12.44 |
| LCE | - | - | - | - | - | - | 61.1 | 1.27 |
| ICI2_H2 | - | 28.8 ± 0.236 | - | 1.22 × 10⁵ ± 5.46 × 10² | - | 3.5 × 10⁻³ ± 2.40 × 10⁻⁵ | 63.0 | 0 |
| SEB7xICI 2_H2 | 7.85 ± 0.173 | 29.9 ± 0.556 | 2.34 × 10⁵ ± 1.56 × 10³ | 1.30 × 10⁵ ± 1.41 × 10³ | 1.84 × 10⁻³ ± 3.87 × 10⁻⁵ | 3.90 × 10⁻³ ± 5.90 × 10⁻⁵ | 65.8 | 10.8 |

### Example 10: CAR-T and/or ADC applications

Treatment with CAR-T-cells is a cellular immunotherapy strategy that uses the patient's own immune system to fight tumours. T-cells are taken from patients and CARs (chimeric antigen receptor) are introduced into them using viral vectors for tumour cell recognition. An antibody fragment that contains two functionalities in a single molecule (2-in-1) and can simultaneously recognise two tumour antigens that are found in large numbers on the cell surface of solid tumors. The binding to solid tumors would be particularly useful.

The ectodomain of the CAR molecule is a key region responsible for the recognition of target antigens. Several strategies for modification and optimisation have been developed, in particular to design antigen recognition in a way that improves the targeting accuracy of CARs. In particular, it has been shown that bispecific CARs that contain two antibody-derived scFv ligand binding domains in tandem and thus simultaneously address two different tumor targets can have an improved efficacy profile (Schneider, Science Transl. Med 2021). Apparently, the increased avidity and specificity of the bispecific CAR improves immunotherapeutic efficacy. This simultaneous targeting also helps to avoid the loss of effect in antigen escape that can occur in heterogeneous solid tumours, as well as to reduce off-target effects. In the case of bispecific tumour cell targeting, it would be desirable to have a binding module that binds both Target A and target B with moderate affinity, but in the AB combination shows a particularly high affinity to tumour cells. The production of such bispecific targeting modules has so far proved difficult. On the one hand, when two scFv molecules are switched in series, it must be ensured that the two scFv domains fold stably and independently of each other and that no light-chain-heavy-chain mixture occurs (so-called chain scrambling), which leads to non-functional molecules. On the other hand, the targeting module should be as small as possible to enable the efficient and productive formation of a cytolytic synapse between the tumour cell and the CAR-T-cell.

To gain access to a bispecific CAR, the VL and VH domains of the Two-in-One antibody are connected through a linker sequence to result in a bifunctional scFv fragment. This fragment is genetically fused to an optional spacer domain, the transmembrane and intracellular domains of an antigen receptor to result in a chimeric antigen receptor. In T-cells, the intracellular signaling domain of the TCR-CD3 complex transduces the necessary signal to kick-start the signaling cascade. Co-stimulatory receptors, especially CD28, are important for sustained signaling, prevention of anergy, and proliferation. In CAR-T-cells, co-stimulatory signals are usually included in-cis with the CD3ζ cytoplasmic domain. The Two-in-One CAR is introduced into T-cells via lentiviral transduction. To this end, CD3+ T-cells, isolated and selected from the Peripheral Blood Mononuclear Cells (PBMC) of healthy donors, are stimulated with anti-CD3/CD28 mAb for T-cell expansion and activation. Cells are transduced with the lentivirus (MOI 5) overnight and transduced cells are expanded. Cell numbers and viability are determined by trypan blue exclusion. To test CAR-T potency, cells are incubated with A431 tumor cells at a tumor cell to CAR-T-cell ratio of 1.25:1 to 10:1 for 72 h. Adherent cells are detached from the plate and stained with anti-CD45 antibodies for T-cell detection and an appropriate antibody for tumor cell detection and analysed by FACS. Alternatively, luciferase-tagged tumor cells are used and the luciferase activity (as indicator of live target cell) is measured in a luminometer. The relative cell viability is calculated by normalizing relative light unit (RLU) from samples against the RLU from target cells without T-cells.

For the Two-in-One antibody, simultaneous engagement of two different targets by a single molecule may have synergistic or emergent therapeutic effects since for ADC, the cancer-selective delivery of potent cytotoxic payloads may eradicate target-expressing cancer cells while sparing normal healthy tissues.

To generate an ADC, a payload is conjugated to the antibody. Among others, one straightforward strategy is to introduce a recognition sequence for microbial transglutaminase to the C-terminus of the Two-in-One antibody heavy or light chain, respectively such as LLQG. A payload consists of a Val-Cit dipeptide cleavable linker to which the toxin MMAE (monomethyauristatin E) is coupled. The payload contains a triple glycine for enzyme-mediated conjugation. For conjugation, the antibody is mixed with the payload in at least two-fold molar excess and microbial transglutaminase is added and incubated for 6 hours. After protein A purification, the conjugate is tested using A431 cells that simultaneously express both targets, i.e. EGFR and PD-L1. This end, the conjugate is added to the cells at various concentrations ranging from 0 to 100 nM followed by incubation of the conjugate with the tumor cells for 72h at 37°C, 5% CO₂. Cell viability is determined using the celltiter 96^{®} aqueous one solution cell proliferation assay (Promega) by recoding absorbance at 490 nm in 96 well plate reader and the IC50 is determined on the basis that the absorbance of the formed formazan is directly proportional to the number of living cells in the culture.

## Claims

1. A bispecific monoclonal antibody or antigen-binding fragment, or a substantially similar variant thereof **characterized in that** at least one Fab-region binds specifically to epidermal growth factor receptor (EGFR) and programmed cell death-ligand 1 (PD-L1).

2. The antibody or antigen-binding fragment or a substantially similar variant thereof according to claim 1, comprising at least one Fᵥ-region binding specifically to epidermal growth factor receptor (EGFR) and programmed cell death-ligand 1 (PD-L1).

3. The antibody or antigen-binding fragment or a substantially similar variant thereof according to any of the previous claims, wherein the paratopes, i.e. the CDR-regions binding to EGFR and PD-L1, on said antibody or antigen-binding fragment or a substantially similar variant thereof do not overlap.

4. The antibody or antigen-binding fragment or a substantially similar variant thereof according to any of the previous claims, capable of inhibiting EGFR-signalling and blocking the PD-1/PD-L1 interaction.

5. The antibody or antigen-binding fragment or a substantially similar variant thereof according to any of the previous claims, wherein the EGFR binding is at the dimerization domain II of EGFR and wherein the antibody prevents binding of PD-L1 to PD-1 and/or competes for binding to PD-L1 with durvalumab.

6. The antibody or antigen-binding fragment or a substantially similar variant thereof according to any of the previous claims, comprising
a heavy chain CDR1 (H-CDR1) domain with an amino acid sequence selected from the group consisting of SEQ ID No.: 03; and/or
a heavy chain CDR2 (H-CDR2) domain with an amino acid sequence selected from the group consisting of SEQ ID No.: 04; and/or
a heavy chain CDR3 (H-CDR3) domain with an amino acid sequence selected from the group consisting of SEQ ID No.: 05; and/or
and/or
a light chain CDR1 (L-CDR1) domain with an amino acid sequence selected from the group consisting of SEQ ID No.: 06; and/or
a light chain CDR2 (L-CDR2) domain with an amino acid sequence selected from the group consisting of SEQ ID No.: 07; and/or
a light chain CDR3 (L-CDR3) domain with an amino acid sequence selected from the group consisting of SEQ ID No.: 08.

7. The antibody or antigen-binding fragment or a substantially similar variant thereof according to any of the previous claims, comprising
a heavy chain V_{H}-region with an amino acid sequence selected from the group consisting of SEQ ID No.: 01;
and/or
a light chain V_{L}-region domain with an amino acid sequence selected from the group consisting of SEQ ID No.: 02.

8. The antibody or antigen-binding fragment or a substantially similar variant thereof according to any of the previous claims, comprising an amino acid sequence at least 95% identical to the amino acid sequence of SEQ ID No.: 01 and/or of SEQ ID No.: 02.

9. The antibody or antigen-binding fragment or a substantially similar variant thereof according to any of the previous claims, comprising a heavy chain variable region of the amino acid sequence in SEQ ID No.: 01 and a light chain variable region of the amino acid sequence in SEQ ID No.: 02.

10. The antibody or antigen-binding fragment or a substantially similar variant thereof according to any of the previous claims, **characterized by** one or more of the following:
- human or humanized, preferably grafted into a human IgG1-scaffold;
- also capable of binding to at least at least a further target protein selected from the group consisting of Fcγ-receptor IIIa /IIIb (CD16a/b), cluster of differentiation 155 (CD155), also known as the poliovirus receptor, MHC class I chain-related proteins A and B (MicA/MicB), cluster of differentiation 159 (CD159), a NKG2 receptor for natural killer cells (NK cells) (there are 7 NKG2 types: A, B, C, D, E, F and H; NKG2D is an activating receptor on the NK cell surface, NKG2A dimerizes with CD94 to make an inhibitory receptor), cluster of differentiation 276 (CD276) or B7 homolog 3 (B7-H3), hepatitis A virus cellular receptor 2 (HAVCR2), also known as T-cell immunoglobulin and mucin-domain containing-3 (TIM-3), T-cell immuno-receptor with Ig and ITIM domains (TIGIT), B-cell maturation antigen (BCMA or BCM), also known as tumor necrosis factor receptor superfamily member 17 (TNFRSF17), interleukin-3 receptor (CD123), natural cytotoxicity triggering receptor 3 (NKp30) or (CD337), natural cytotoxicity triggering receptor 3 (NKp44) or (CD336), natural cytotoxicity triggering receptor 3 (NKp46) or (CD335), cluster of differentiation 27 (CD27), cluster of differentiation 96 (CD96) or Tactile (T-cell activation, increased late expression), NKRP1A, cluster of differentiation 355 (CD355) or Cytotoxic and regulatory T-cell molecule (CRTAM), and lymphocyte function-associated antigen 1 (LFA-1), and/or any combination thereof;
- capable of binding specifically to PD-L1 -protein with an affinity (KD) of between 50 and 500 nM;
- capable of binding specifically to EGFR -protein with an affinity (KD) of 150 and 1000 nM;
- capable to specifically bind to a tumour cell, wherein the tumour cell is **characterized by** being double-positive for PD-L1 and EGFR;
- inhibits the dimerization of EGFR, but not the binding of EGF to EGFR;
- inhibits EGFR-pathway signalling at least 2-fold or more;
- inhibits the binding of PD-L1 to PD-1 with an EC₅₀ of between 5 and 500 nM;
- binds to double-positive cells with EGFR and PD-L1 with an EC₅₀ of between 0.5 and 20 nM;
- being further modified to an antibody-drug conjugate (ADC) by being conjugated to a therapeutic agent, such as drug, a tumour-growth inhibitor, an apoptotic signal molecule, a toxin or the like;
- being further modified to extend its half-life by albumin-fusion, polyaminoacid fusion, fc fusion, or a polymer-conjugation with polyethylene glycol, sialic acid, starch and/or hyaluronic acid.

11. An isolated nucleic acid molecule encoding the bispecific monoclonal antibody or antigen-binding fragment, or a substantially similar variant thereof according to any one of the previous claims.

12. An expression vector comprising the nucleic acid molecule according to claim 11.

13. A method of producing the antibody or antigen-binding fragment or a substantially similar variant thereof according to any one of the previous claims comprising the steps of
i. Selecting a first and a second human target protein, preferably represented on the surface of tumour cells, preferably EGFR and PD-L1;
ii. Creating a first yeast surface display (YSD) library, comprising the following steps:
a. Immunize an animal of avian species, preferably a chicken, with the first target protein;
b. Isolate variable heavy chain (VH) genes from cDNA derived from said immunized animal of avian species;
c. Insert the VH genes into a vector designed for expression, secretion, and display of proteins on the extracellular surface of *Saccharomyces cerevisiae* cells, preferably pYD1-derived, and transform yeast cells with said vector;
d. Insert genes for a common variable light chain (VL) (SEQ ID No.: 16) into an expression vector designed for expression, secretion, and display of proteins on the extracellular surface of *Saccharomyces cerevisiae* cells, preferably pYD1-derived and transform yeast cells with said vector;
e. Combine the two yeast cells by yeast cell mating, resulting in diploid yeast cells displaying VH domains from the PD-L1-immunized animal of avian species, paired with the common variable light chain (VL) (SEQ ID No.: 16);
f. Screen by fluorescence activated cell sorting (FACS) for the binding to the first target protein and isolate yeast cells displaying antibodies which specifically bind to the first target protein;
iii. Creating a second yeast surface display (YSD) library, comprising the following steps:
a. Immunize an animal of avian species, preferably a chicken, with the second target protein; preferably EGFR if the first antigen was PD-L1, or PD-L1 if the first antigen was EGFR;
b. Isolate variable light chain (VL) genes from cDNA derived from said immunized animal of avian species;
c. Insert genes for the common variable heavy chain (VH) gene (SEQ ID No.: 15) into an vector designed for expression, secretion, and display of proteins on the extracellular surface of *Saccharomyces cerevisiae* cells, preferably pYD1-derived, and transform yeast cells with said vector;
iv. Combining the yeast cells from the first YSD-library carrying heavy chains (VH) specifically binding to the first target protein with the second YSD-library carrying the light chains (VL) from the animal of avian species immunized with the second target protein, by yeast mating
v. Screening the resulting yeast cells by fluorescence activated cell sorting (FACS) for binding to both target proteins; and/or inhibition of each of the two target proteins;
vi. Identifying the yeast cells comprising antibodies comprising heavy chains specifically binding to the first target protein and comprising light chains specifically binding to the second target protein;
vii. Isolating the bispecific, tetravalent humanized or a human monoclonal antibody or antigen-binding fragment binding to both target proteins, preferably EGFR and PD-L1,
viii. Receiving the antibody or antigen-binding fragment or a substantially similar variant thereof according to any one of the previous claims.

14. A pharmaceutical composition comprising the bispecific monoclonal antibody or antigen-binding fragment, or a substantially similar variant thereof according to any one of claims 1 to 10, and a pharmaceutically acceptable carrier.

15. A bispecific monoclonal antibody or antigen-binding fragment, or a substantially similar variant thereof according to any one of claims 1 to 10 for the use in the identification, prevention and/or treatment of a disease or condition in a patient.
